# EUROPEAN PATENT SPECIFICATION

(11) **EP 2 091 939 B1**
(45) Date of publication and mention of the grant of the patent: **16.03.2011**
(21) Application number: 07857792.1
(22) Date of filing: 19.12.2007
(51) Int. Cl.: C07D 401/12, A61K 31/473, A61P 25/28, A61P 25/30, A61P 25/24

(54) **COMPOUNDS WITH A COMBINATION OF CANNABINOID-CB1 ANTAGONISM AND ACETYLCHOLINESTERASE INHIBITION**
VERBINDUNGEN MIT KOMBINIERTEN CANNABINOID-CB1-ANTAGONISMUS UND ACETYLCHOLINESTERASEHEMMUNG
COMPOSÉS PRÉSENTANT UNE COMBINAISON D'ANTAGONISME DE CANABINOÏDE-CB1 ET D'INHIBITION D'ACÉTYLCHOLINESTÉRASE

(30) Priority: 20.12.2006 EP 06126584; 20.12.2006 US 875808 P
(43) Date of publication of application: 26.08.2009
(73) Proprietor: Abbott Healthcare Products B.V., 1381 CP Weesp (NL)
(72) Inventor: LANGE, Josephus H.M., 1381 CP Weesp (NL); KRUSE, Cornelis G., 1381 CP Weesp (NL); SHADID, Belal, 1381 CP Weesp (NL)
(74) Representative: Tulp, Martinus
(86) International application number: PCT/EP2007/064169
(87) International publication number: WO 2008/074816

(56) References cited:
- WO-A-2005/037199
- WO-A-2005/118553
- LANGE J H M ET AL: "SYNTHESIS, BIOLOGICAL PROPERTIES, AND MOLECULAR MODELING INVESTIGATIONS OF NOVEL 3,4-DIARYLPYRAZOLINES AS POTENT AND SELECTIVE CB1 CANNABINOID RECEPTOR ANTAGONISTS" JOURNAL OF MEDICINAL CHEMISTRY, AMERICAN CHEMICAL SOCIETY, US, vol. 47, no. 3, 2004, pages 627-643, XP001188902 ISSN: 0022-2623 cited in the application
- CARLIER P R ET AL: "Heterodimeric tacrine-based acetylcholinesterase inhibitors: investigating ligand-peripheral site interactions." JOURNAL OF MEDICINAL CHEMISTRY 7 OCT 1999, vol. 42, no. 20, 7 October 1999 (1999-10-07), pages 4225-4231, XP002434602 ISSN: 0022-2623 cited in the application
- MORPHY RICHARD ET AL: "Designed multiple ligands. An emerging drug discovery paradigm." JOURNAL OF MEDICINAL CHEMISTRY 20 OCT 2005, vol. 48, no. 21, 20 October 2005 (2005-10-20), pages 6523-6543, XP002434603 ISSN: 0022-2623

## Description

### TECHNICAL FIELD

This invention relates to the fields of pharmaceutical and organic chemistry, and provides compounds with a combination of cannabinoid-CB₁ antagonism and cholinesterase inhibition, intermediates, formulations and methods.

### BACKGROUND ART

A reductionist 'one target-one disease' approach dominates the pharmaceutical industry for some decades. Using this strategy, many successful drugs were discovered. Despite that however, many diseases remain inadequately treated. These findings rationalize an alternative approach, wherein chemical entities are developed that simultaneously modulate multiple targets. Such drugs may show advantageous properties such as increased clinical efficacy, or lack of undesired pharmacokinetic drug-drug interactions, or unfavourable pharmacokinetic and pharmacodynamic properties. The latter may lead to unpredictable variability between individual patients. In order to combine different therapeutic mechanisms, cocktails of two or more drugs are still used in clinical practice. Alternatively, multicomponent drugs are being used wherein two or more pharmaceutically active compounds are coformulated in a single tablet or capsule in order to improve patient compliance. Another approach utilizes a pharmaceutical treatment with a chemical entity that is able to modulate more than one biological target simultaneously. It is clear that such a 'single entity-multiple target approach' offers the advantage of a lower risk of undesired drug-drug interactions compared to drug cocktails or multicomponent drugs. Several multiple target ligands are known. The majority were found retrospectively or by accident: Only a few were rationally designed.

Cannabinoid receptors are part of the endo-cannabinoid system, involved in many diseases. Detailed information on cannabinoid receptors, CB₁ receptor modulators, and their pharmacological activities, are subjects of recent reviews *(Landsman, 1997*; *Lichtman, 2002; De Petrocellis, 2004; Di Marzo, 2004; Hertzog, 2004; Lange, 2004, 2005; Smith, 2005; Thakur, 2005; Padgett, 2005; Muccioli, 2005; Lambert, 2005; Vandevoorde, 2005).* Potential therapeutic applications of CB₁ receptor modulators disclosed in the quoted reviews include medicaments for treating psychosis, anxiety, depression, attention deficits, memory disorders, cognitive disorders, appetite disorders, obesity, addiction, appetence, drug dependence, neurodegenerative disorders, dementia, dystonia, muscle spasticity, tremor, epilepsy, multiple sclerosis, traumatic brain injury, stroke, Parkinson's disease, Alzheimer's disease, epilepsy, Huntington's disease, Tourette's syndrome, cerebral ischaemia, cerebral apoplexy, craniocerebral trauma, spinal cord injury, neuroinflammatory disorders, plaque sclerosis, viral encephalitis, demyelinisation related disorders, as well as for the treatment of pain disorders, including neuropathic pain disorders, septic shock, glaucoma, diabetes, cancer, emesis, nausea, gastrointestinal disorders, gastric ulcers, diarrhoea, sexual disorders, impulse control disorders and cardiovascular disorders.

Cholinesterases, including acetylcholinesterase and butyrylcholinesterase, are serine hydrolases. Alzheimer's disease (AD) is a neurodegenerative disorder whose prevalence is increasing together with life expectancy throughout the world. Cholinergic-enhancing drugs are the main current therapy *(Terry, 2003).* Tacrine (Trade name: Cognex^{®}) was the first approved by the FDA for AD treatment. This substance, an acetylcholinesterase as well as a butyrylcholinesterase inhibitor, showed clinically significant improvement in cognition in AD-patients. Currently three other AChE inhibitors are available for treating AD: donepezil (Aricept^{®}), rivastigmine (Exelon^{®}), and galanthamine (Reminyl^{®}). Like donezepil and rivastigmine, tacrine is a reversible inhibitor that presumably acts centrally by elevating the acetylcholine level in the cerebral cortex, and by slowing degradation of acetylcholine from intact cholinergic neurons *(Brufani, 1997*; *Weinstock, 1999)*. It has been shown that AChE inhibitors have potential in the modulation of amyloid precursor protein processing *(Racchi, 2004).* The structures of tacrine, amiridine, 7-methoxytacrine and SM-10888 are closely related, whereas for example donezepil has a somewhat more elongated structure. Structures and cholinesterase inhibiting activities of compounds structurally related to tacrine were recently reviewed *(Marco, 2003),* but many more AChE inhibitors have been described in the (patent) literature.

Drug dependence poses serious social, medical, and economic problems. Effective treatments are still limited. Recently, it was found that AChE inhibitors that act on the brain suppressed both cocaine- and morphine-induced conditioned place preference, and blocked the induction and persistence of cocaine-evoked hyperlocomotion. Thus, centrally active AChE inhibitors are novel potential therapeutic agents for drug addiction *(Hikida, 2003).* Also cannabinoid CB₁ antagonists were suggested for treating drug addiction *(Cohen, 2002; Hungund, 2002; Solinas, 2003).* AChE inhibitors have shown efficacy not only in Alzheimer's disease *(Spencer, 1998)*, but also in other cognitive disorders such as dementia with Lewy bodies *(McKeith, 2000),* Parkinson's disease *(Werber, 2001),* vascular dementia *(Kumar, 2000),* and traumatic brain injury *(Masanic, 2001).* Butyryl-cholinesterase is considered as a potential target for Alzheimer's disease because it also regulates acetylcholine levels *(Darvesh, 2003).*

Cognitive disorders are also a potential therapeutic area for cannabinoid CB₁ receptor antagonists *(Castellano, 2003; Wolff, 2003).* CB₁ receptor antagonists were shown to increase acetylcholine (Ach) release in certain brain areas including the cortical region and hippocampus *(De Groot, 2006*). The selective CB₁ receptor antagonist rimonabant showed neuroprotective activity in animal stroke models *(Berger, 2004).* In summary, scientific articles, patents and patent applications indicate the following therapeutic applications for cholinesterase inhibitors: alcoholism, Alzheimers disease, amnesia, arthritis, cancer, central nervous system disease, cognitive disorder, constipation, dementia, dyspepsia, gastric motility disorder, gastrointestinal disease, gastroparesis, glaucoma, irritable bowel syndrome, major depressive disorder, migraine, multiple sclerosis, muscle disease, muscular dystrophy, myasthenia gravis, neurodegenerative disease, neuropathic pain, nicotine dependence, *Pediculus capitis* infestation, poison intoxication, postviral fatigue syndrome, psychiatric disorder, senile dementia, schistosomiasis, urinary dysfunction and xerostomia.

Because of the frequently observed co-morbidity of symptoms of different diseases, compounds combining cannabinoid-CB₁ antagonism with cholinesterase inhibition can be useful to treat the conditions wherein either a cannabinoid CB₁ antagonist or a cholinesterase inhibitor is potentially effective. Thus the compounds of the invention can be used for treating addiction, appetence, alcoholism, Alzheimers disease, amnesia, anxiety, appetite disorders, arthritis, attention deficits, cancer, cardiovascular disorders, central nervous system disease, cerebral apoplexy, cerebral ischaemia, cognitive disorder, constipation, dementia, demyelinisation related disorders, depression, diabetes, diarrhoea, drug dependence, dyspepsia, dystonia, emesis, epilepsy, gastric motility disorder, gastric ulcers, gastrointestinal disorders, gastroparesis, glaucoma, Huntington's disease, impulse control disorders, irritable bowel syndrome, memory disorders, migraine, multiple sclerosis, muscle disease, muscular dystrophy, muscle spasticity, myasthenia gravis, nausea, neurodegenerative disorders, neuroinflammatory disorders, neuropathic pain, nicotine dependence, obesity, pain disorders, Parkinson's disease, *Pediculus capitis* infestation, plaque sclerosis, poison intoxication, postviral fatigue syndrome, psychiatric disorder, psychosis, senile dementia, septic shock, sexual disorders, schistosomiasis, spinal cord injury, stroke, Tourette's syndrome, traumatic brain injury, tremor, urinary dysfunction, viral encephalitis and xerostomia.

Of particular importance is the use of the compounds of the invention for treating disorders that are claimed to be treatable with cannabinoid CB₁ antagonists as well as with cholinesterase inhibitors. Attacking such disorders simultaneously via two different mechanisms of action can have synergistic effects. The compounds of the invention are particularly useful for treating Alzheimer's disease, cognitive disorders, memory disorders, dementia, attention deficits, traumatic brain injury, drug dependence, addiction and substance abuse.

The pharmacophore of the majority of cannabinoid CB₁ receptor antagonists was the subject of several reviews *(Lange, 2005; Reggio, 2003).* Scheme 1 visualizes it. 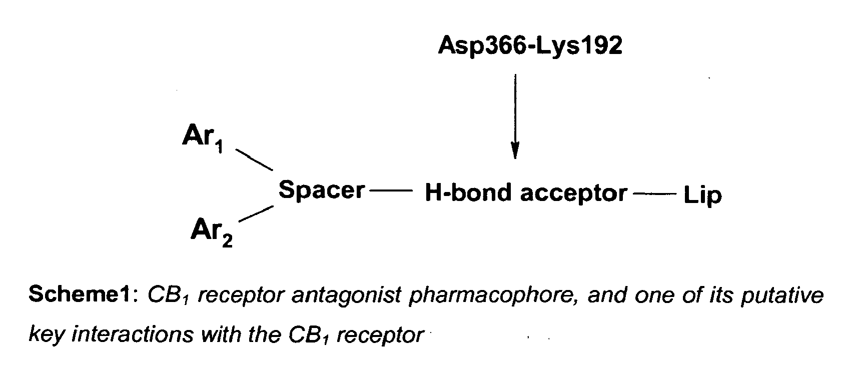 In Scheme 1 Ar₁ and Ar₂ represent phenyl groups, optionally substituted with one or two halogen atoms, trifluoromethyl groups, or methoxy groups. The 'spacer' contains a five-membered heterocyclic group such as 4,5-dihydropyrazole, imidazole, pyrazole, thiazole, thiophene, or pyrrole or the spacer contains a phenyl group or a six-membered heterocyclic group such as pyridine, pyrimidines or pyrazine. The spacer can also contain a azetidine moiety, a 1,3-benzodioxole moiety or an alkyl moiety like in MK-0364 (see below). In addition, one of the aromatic groups can be fused to the spacer, or can be connected to the spacer by an additional ring: so-called conformational constraint. Several kinds of conformational constraints have successfully been implemented in this pharmacophore model. The H-bond acceptor represents a carbonyl group, a sulfonyl group or a nitrogen-atom which might be embedded in a heterocyclic ring structure such as an imidazole ring. In Scheme 1, 'Lip' represents a lipophilic moiety, for instance piperidin-1-ylamino, pyrrolidinyl-1-amino, cycloalkylamino, phenylamino, arylamino, benzyl-amino or alkylamino.

Molecular modeling studies indicate that the presence of a hydrogen bond acceptor is crucial: that is thought to interact with the Lys-192 amino acid residue side chain in the CB₁ receptor, thereby stabilizing its inactive state. To illustrate the CB₁ receptor antagonist pharmacophore model, a number of concrete examples of CB₁ receptor antagonists are depicted in below. The putative hydrogen bond acceptor atom (oxygen atom from a carbonyl group, oxygen atom from a sulfonyl group, or N atom in a heteroaromatic ring) in the CB₁ receptor antagonists are indicated in **bold:**

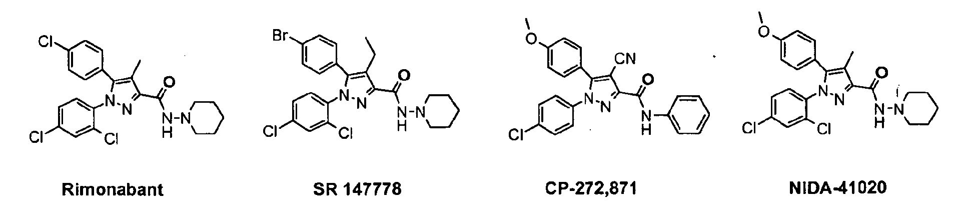

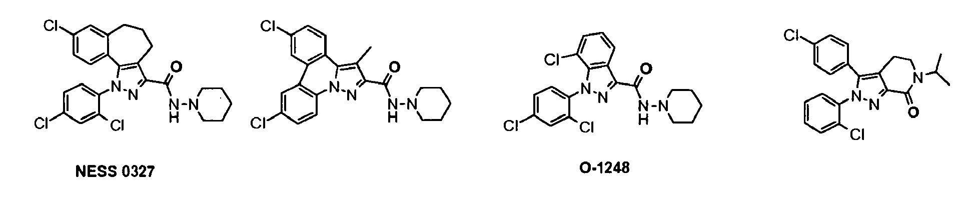

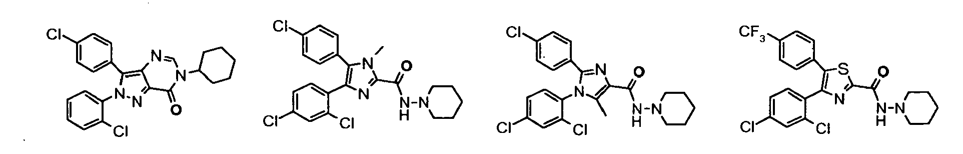

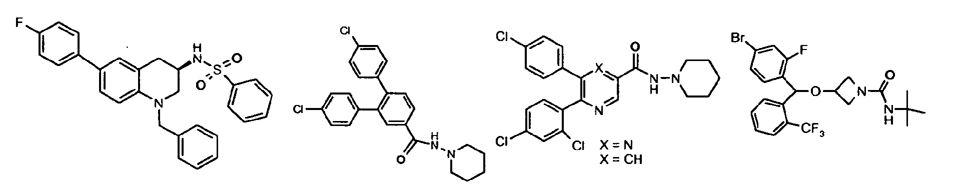

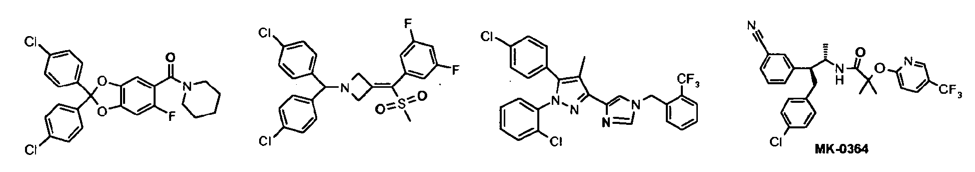

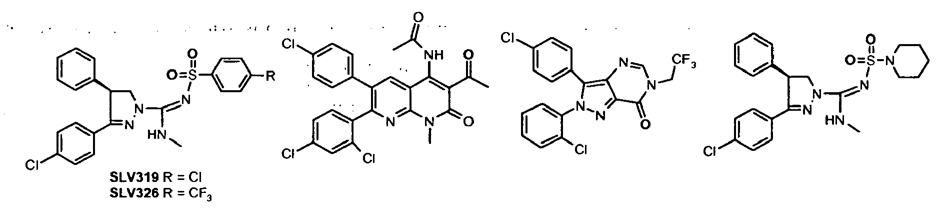

The selective CB₁ receptor antagonist SR141716A (rimonabant) is known for more than a decade. Many other selective CB₁ receptor antagonists were invented later. Several acetylcholinesterase inhibitors are known for many years, too. For example, tacrine was approved in the USA in 1993. No compounds have hitherto been disclosed which exhibit a combination of CB₁ receptor antagonist and acetylcholinesterase inhibitor activities.

The objective of the present invention was to develop compounds with a combination of CB₁ antagonism and acetylcholinesterase inhibition.

### DISCLOSURE

It was found that molecules containing essential parts of known cannabinoid-CB₁ antagonists and essential parts of the known acetylcholine esterase inhibitor tacrine, share the activity of both molecules from which they were derived: cannabinoid-CB₁ antagonism and inhibition of acetylcholine esterase.

This invention concerns compounds with a combination of CB₁ antagonism and acetyl- and/or butyrylcholinesterase inhibition. Particularly, this invention concerns compounds of formula (1): 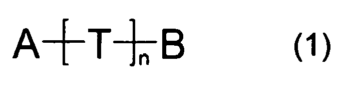 or a tautomer, stereoisomer, N-oxide, isotopically-labelled analogue, or a pharmacologically acceptable salt, hydrate or solvate of any of the foregoing, wherein:
A represents one of the fragments (A^{1a}), (A^{1b}), (A²), (A³) (A⁴), (A⁵), (A⁶), (A⁷), or (A⁸): 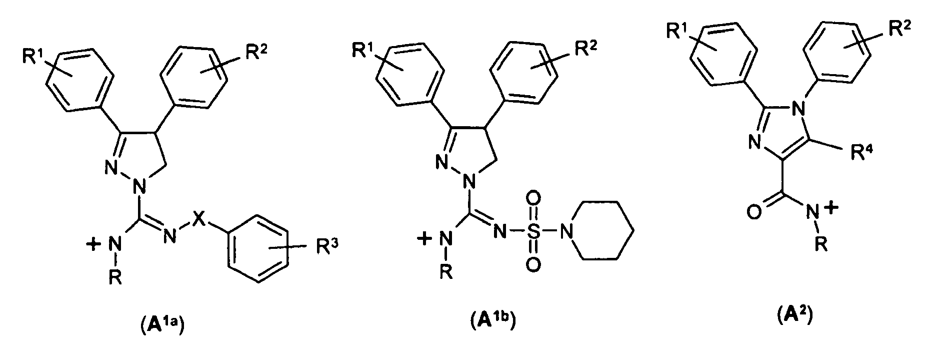 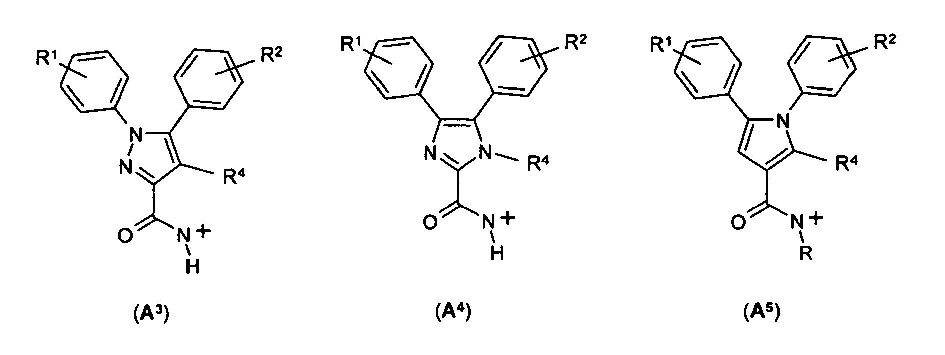 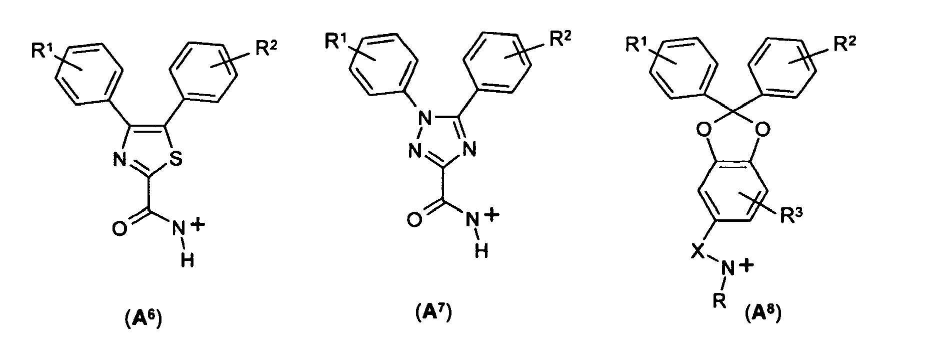 wherein, X represents a sulfonyl or a carbonyl group, the "+" symbol represents the point at which the fragment is attached to the linker T of formula (1), R¹, R² and R³ independently represent or more hydrogen atoms, trifluoromethyl groups or halogen atoms, R⁴ represents a hydrogen or halogen atom, or a methyl, ethyl, trifluoromethyl, hydroxymethyl, fluoromethyl, 2,2,2-trifluoroethyl, propyl, methylsulfanyl, methylsulfinyl, methylsulfonyl, ethylsulfanyl, ethylsulfinyl, ethylsulfonyl, C₁₋₃-dialkyl-aminomethyl, pyrrolidin-1-ylmethyl, piperidin-1-ylmethyl or morpholin-4-ylmethyl group, and wherein R represents a hydrogen atom or a C₁₋₃ alkyl group.
- **T** represents a linker consisting of a saturated or unsaturated linear carbon chain of 2-8 atoms, which carbon chain may be substituted with 1-5 substituents selected from methyl, ethyl, hydroxy, fluoro or amino, which carbon chain may contain an additional nitrogen atom, optionally substituted with a C₁₋₃ alkyl group, or which carbon chain may contain an additional oxygen or sulphur atom, or a carbonyl, sulfonyl, amide, sulfonamide, ureido, or aryl group, which aryl group is optionally substituted with 1-4 substituents selected from the group consisting of halogen, cyano, methyl, methoxy, trifluoromethyl, OCHF₂, OCF₃, SCF₃ or nitro,
- B is chosen from one of the fragments (B¹), (B²) or (B³)_{:} 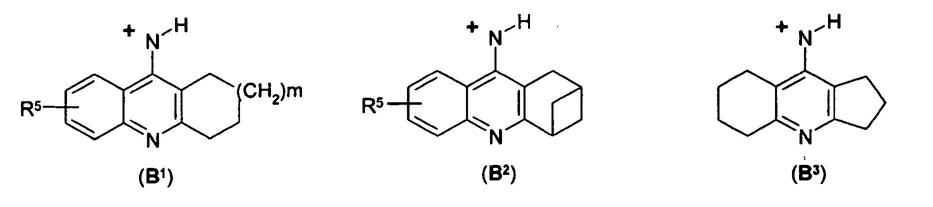 wherein, the "+" symbol represents the point at which the fragment is attached to the linker T of formula (1), R⁵ represents a hydrogen or a halogen atom, or a methoxy or a trifluoromethoxy group, and m is an integer which can have the value 0, 1 or 2,
- n = 0 or 1

In another embodiment the invention relates to compounds of formula (1) in which A represents one of the fragments (A^{1a}), (A^{1b}) or (A²), said acetylcholine esterase inhibitor of fragment B is tacrine and the other symbols have the meanings as given above.

In another embodiment the invention relates to compounds of formula (1) in which A represents one of the fragments (A⁹) or (A¹⁰): 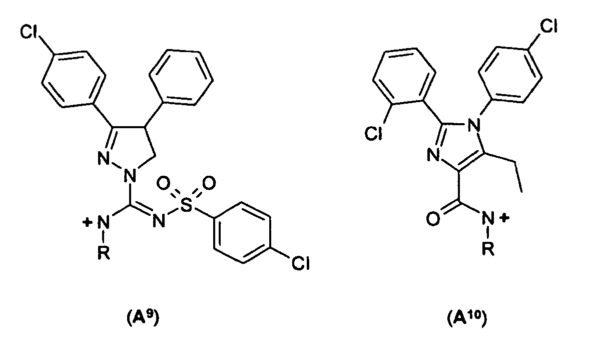 and the other symbols have the meanings as given above.

in another embodiment the invention relates to the compounds of formula (1) that are:

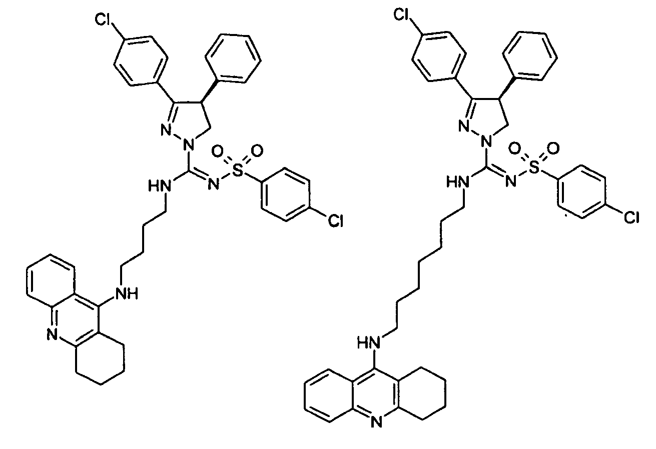

The compounds of formula (1), as well as tautomers, stereoisomers, N-oxides, isotopically-labelled analogues thereof, and pharmacologically acceptable salts, hydrates and solvates of any of the foregoing, have a combination of cannabinoid-CB₁ antagonism and cholinesterase inhibition, in particular acetylcholinesterase inhibition. They are useful in the treatment of disorders in which cannabinoid-CB₁ receptors and acetylcholine esterase sites are involved, or that can be treated via modulation of those receptors such as addiction, appetence, alcoholism, Alzheimers disease, amnesia, anxiety, appetite disorders, arthritis, attention deficits, cancer, cardiovascular disorders, central nervous system disease, cerebral apoplexy, cerebral ischaemia, cognitive disorder, constipation, dementia, demyelinisation related disorders, depression, diabetes, diarrhoea, drug dependence, dyspepsia, dystonia, emesis, epilepsy, gastric motility disorder, gastric ulcers, gastrointestinal disorders, gastroparesis, glaucoma, Huntington's disease, impulse control disorders, irritable bowel syndrome, memory disorders, migraine, multiple sclerosis, muscle disease, muscular dystrophy, muscle spasticity, myasthenia gravis, nausea, neurodegenerative disorders, neuroinflammatory disorders, neuropathic pain, nicotine dependence, obesity, pain disorders, Parkinson's disease, *Pediculus capitis* infestation, plaque sclerosis, poison intoxication, postviral fatigue syndrome, psychiatric disorder, psychosis, senile dementia, septic shock, sexual disorders, schistosomiasis, spinal cord injury, stroke, Tourette's syndrome, traumatic brain injury, tremor, urinary dysfunction, viral encephalitis and xerostomia.

Other embodiments of the invention include, but are not limited to:
a pharmaceutical composition for treating, for example, a disorder or condition that may be treated by a combination of cannabinoid-CB₁ antagonism and acetylcholine esterase inhibition, the composition comprising a compound of formula(1), and a pharmaceutically acceptable carrier;
a pharmaceutical composition for treating, for example, a disorder or condition selected from the group consisting of the disorders listed herein;
a pharmaceutical composition for treating a disorder or condition selected from the group consisting of the disorders listed herein, the composition comprising a compound of formula(1), and a pharmaceutically acceptable carrier;

The invention also provides the use of a compound according to formula (1) for the manufacture of a medicament.

The invention further relates to combination therapies wherein a compound of the invention, or a pharmaceutical composition or formulation comprising a compound of the invention, is administered concurrently or sequentially or as a combined preparation with another therapeutic agent or agents, for the treatment of one or more of the conditions listed. Such other therapeutic agent(s) may be administered prior to, simultaneously with, or following the administration of the compounds of the invention.

The invention also provides compounds, pharmaceutical compositions, kits and methods for treating a disorder or condition selected from the group consisting of the disorders listed herein, the method comprising administering to a patient in need of such treatment a compound of formula (1).

The compounds of the invention possess combination of cannabinoid-CB₁ antagonism and cholinesterase inhibition, in particular acetylcholine esterase inhibition. The (ant)agonizing /inhibiting activities of the compounds of the invention is readily demonstrated, for example, using one or more of the assays described herein or known in the art.

The invention also provides methods of preparing the compounds of the invention and the intermediates used in those methods.

Isolation and purification of the compounds and intermediates described herein can be affected, if desired, by any suitable separation or purification procedure such as, for example, filtration, extraction, crystallization, column chromatography, thin-layer chromatography, thick-layer chromatography, preparative low or high-pressure liquid chromatography, or a combination of these procedures. Specific illustrations of suitable separation and isolation procedures can be taken from the preparations and examples. However, other equivalent separation or isolation procedures could, of course, also be used.

The compounds of the present invention may contain one or more asymmetric centers and can thus occur as racemates and racemic mixtures, single enantiomers, diastereomeric mixtures and individual diastereomers. Additional asymmetric centers may be present depending upon the nature of the various substituents on the molecule. Each such asymmetric center will independently produce two optical isomers and it is intended that all of the possible optical isomers and diastereomers in mixtures and as pure or partially purified compounds are included within the ambit of this invention. The present invention is meant to comprehend all such isomeric forms of these compounds. Formula (1) shows the structure of the class of compounds without preferred stereochemistry. The independent syntheses of these diastereomers or their chromatographic separations may be achieved as known in the art by appropriate modification of the methodology disclosed herein. Their absolute stereochemistry may be determined by the x-ray crystallography of crystalline products or crystalline intermediates which are derivatized, if necessary, with a reagent containing an asymmetric center of known absolute configuration. If desired, racemic mixtures of the compounds may be separated so that the individual enantiomers are isolated. The separation can be carried out by methods well known in the art, such as the coupling of a racemic mixture of compounds to an enantiomerically pure compound to form a diastereomeric mixture, followed by separation of the individual diastereomers by standard methods, such as fractional crystallization or chromatography. The coupling reaction is often the formation of salts using an enantiomerically pure acid or base, such as for example (-)di-p-toluoyl-D-tartaric acid and/or (+)-di-p-toluoyl-L-tartaric acid The diasteromeric derivatives may then be converted to the pure enantiomers by cleavage of the added chiral residue. The racemic mixture of the compounds can also be separated directly by chromatographic methods utilizing chiral stationary phases, which methods are well known in the art. Alternatively, any enantiomer of a compound may be obtained by stereoselective synthesis using optically pure starting materials or reagents of known configuration by methods well known in the art.

Cis and trans isomers of the compound of formula (1) or a pharmaceutically acceptable salt thereof are also within the scope of the invention, and this also applies to tautomers of the compounds of formula (1) or a pharmaceutically acceptable salt thereof.

Some of the crystalline forms for the compounds may exist as polymorphs and as such are intended to be included in the present invention. In addition, some of the compounds may form solvates with water (i.e., hydrates) or common organic solvents, and such solvates are also intended to be encompassed within the scope of this invention.

Isotopically-labeled compound of formula (1) or pharmaceutically acceptable salts thereof, including compounds of formula (1) isotopically-labeled to be detectable by PET or SPECT, are also included within the scope of the invention, and same applies to compounds of formula (1) labeled with [¹³C]-, [¹⁴C]-, [¹⁸F]-, [³H]-, [¹²⁵I]- or other isotopically-enriched atoms, suitable for receptor binding or metabolism studies.

The compounds of the invention may also be used as reagents or standards in the biochemical study of neurological function, dysfunction and disease.

### DEFINITIONS

Within the context of this description, the terms **compound with cannabinoid-CB₁ antagonism'** and **cannabinoid-CB₁ antagonist'** refer to compounds having this activity-*measured by unambiguous and well accepted pharmacological assays, including those described herein*-without displaying substantial cross-reactivity towards another receptor. In one embodiment, a compound of the present invention is at least 10 times more potent as cannabinoid-CB₁ antagonist than as agonist or antagonist on any other receptor. Preferred are compounds with a 100-fold selectivity, most preferred are compounds with a selectivity of a factor 1,000 or higher, he term **"compound with cholinesterase inhibiting acitivity"** or **cholinesterase inhibitor'** refer to a compounds having this activity*-measured by unambiguous and well accepted pharmacological assays, including those described herein-*without displaying substantial cross-reactivity towards another receptor. In one embodiment, a compound of the present invention is at least 10 times more potent as cholinesterase inhibitor than as inhibitor of any other enzyme. Preferred are compounds with a 100-fold selectivity, most preferred are compounds with a selectivity of a factor 1,000 or more. A compound **"having both cannabinoid-CB₁ antagonism and cholinesterase inhibiting activity",** refers to compounds having both activities-measured *by unambiguous and well accepted pharmacological assays, including those described herein*-without displaying substantial cross-reactivity towards other receptors or enzymes. In one embodiment, a compound of the present invention is at least 10 times more potent as cannabinoid-CB₁ antagonist and as cholinesterase inhibitor, than as agonist or antagonist on any other receptor or as inhibitor of any other enzyme. Preferred are compounds with a 100-fold selectivity, most preferred are compounds with a selectivity of a factor 1,000 or more.

General terms used in the description of compounds herein disclosed bear their usual meanings. The term **alkyl** as used herein denotes a univalent saturated, branched or straight, hydrocarbon chain. Unless otherwise stated, such chains can contain from 1 to 18 carbon atoms. Representative of such alkyl groups are methyl, ethyl, propyl, isopropyl, butyl, isobutyl, sec-butyl, fert-butyl, pentyl, isopentyl, neopentyl, tert-pentyl, hexyl, isohexyl, heptyl, octyl, nonyl, decyl, undecyl, dodecyl, tridecyl, tetradecyl, pentadecyl, hexadecyl, heptadecyl, octadecyl, and the like. When qualified 'lower', the alkyl group will contain from 1 to 6 carbon atoms. The same carbon content applies to the parent term 'alkane', and to derivative terms such as 'alkoxy'. The carbon content of various hydrocarbon containing moieties is indicated by a prefix designating the minimum and maximum number of carbon atoms in the moiety, i.e., the prefix Cₓ-C_{y} defines the number of carbon atoms present from the integer "x" to the integer "y" inclusive. **'Alkyl(C₁₋₃)'** for example, means methyl, ethyl, n-propyl or isopropyl, and **'alkyl(C₁₋₄)'** means 'methyl, ethyl, n-propyl, isopropyl, n-butyl, 2-butyl, isobutyl or 2-methyl-n-propyl'.

The term **'acyl'** means alkyl(C₁₋₃) carbonyl, arylcarbonyl or aryl-alkyl(C₁₋₃)carbonyl. **'Aryl'** embraces monocyclic or fused bicyclic aromatic or hetero-aromatic groups, including but not limited to furyl, thienyl, pyrrolyl, oxazolyl, thiazolyl, imidazolyl, imidazo[2,1-b][1,3]thiazolyl, pyrazolyl, isoxazolyl, isothiazolyl, pyridyl, pyridazinyl, pyrimidinyl, pyrazinyl, 1,3,5-triazinyl, phenyl, indazolyl, indolyl, indolizinyl, isoindolyl, benzo[b]furanyl, 1,2,3,4-tetrahydro-naphtyl, 1,2,3,4-tetrahydroisoquinolinyl, indanyl, indenyl, benzo[b]thienyl, 2,3-dihydro-1,4-benzodioxin-5-yl, benzimidazolyl, benzothiazolyl, benzo[1,2,5]thia-diazolyl, purinyl, quinolinyl, isoquinolinyl, phtalazinyl, quinazolinyl, quinoxalinyl, 1,8-naphthyridinyl, naphthyl, pteridinyl or azulenyl. **'Halo'** or **'Halogen'** means chloro, fluoro, bromo or iodo; **'hetero'** as in 'heteroalkyl, heteroaromatic' etc. means containing one or more N, O or S atoms. **'heteroalkyl'** includes alkyl groups with heteroatoms in any position, thus including N-bound O-bound or S-bound alkyl groups.

The term **"substituted"** means that the specified group or moiety bears one or more substituents. Where any group may carry multiple substituents, and a variety of possible substituents is provided, the substituents are independently selected, and need not to be the same. The term **"unsubstituted"** means that the specified group bears no substituents. With reference to substituents, the term **"independently"** means that when more than one of such substituents are possible, they may be the same or different from each other.

The terms **"oxy", "thio"** and **"carbo"** as used herein as part of another group respectively refer to an oxygen atom, a sulphur atom and a carbonyl (C=O) group, serving as linker between two groups, such as for instance hydroxyl, oxyalkyl, thioalkyl, carboxyalkyl, etc. The term **"amino"** as used herein alone, or as part of another group, refers to a nitrogen atom that may be either terminal, or a linker between two other groups, wherein the group may be a primary, secondary or tertiary (two hydrogen atoms bonded to the nitrogen atom, one hydrogen atom bonded to the nitrogen atom and no hydrogen atoms bonded to the nitrogen atom, respectively) amine. The terms **"sulfinyl"** and **"sulfonyl"** as used herein as part of another group respectively refer to an -SO- or an - SO₂- group.

To provide a more concise description, the terms **'compound' or 'compounds'** include tautomers, stereoisomers, N-oxides, isotopically-labelled analogues, or pharmacologically acceptable salts, hydrates or solvates, also when not explicitly mentioned.

As used herein, the term **"leaving group"** (L) shall mean a charged or uncharged atom or group that departs during a substitution or displacement reaction. The term refers to groups readily displaceable by a nucleophile, such as an amine, a thiol or an alcohol nucleophile. Such leaving groups are well known in the art. Examples include, but are not limited to, N-hydroxysuccinimide, N-hydroxybenzotriazole, halides (Br, Cl, I), triflates, mesylates, tosylates, and the like.

**N-oxides** of the compounds mentioned above belong to the invention. Tertiary amines may or may not give rise to N-oxide metabolites. The extent to what N-oxidation takes place varies from trace amounts to a near quantitative conversion. N-oxides may be more active than their corresponding tertiary amines, or less active. Whilst N-oxides can easily be reduced to their corresponding tertiary amines by chemical means, in the human body this happens to varying degrees. Some N-oxides undergo nearly quantitative reductive conversion to the corresponding tertiary amines, in other cases conversion is a mere trace reaction, or even completely absent (*Bickel, 1969*).

Any compound metabolized *in vivo* to provide the bioactive agent (i.e., the compound of formula (1)) is a **prodrug**. Prodrugs are therapeutic agents, inactive per se but transformed into one or more active metabolites. Prodrugs are bioreversible derivatives of drug molecules used to overcome some barriers to the utility of the parent drug molecule. These barriers include, but are not limited to, solubility, permeability, stability, presystemic metabolism and targeting limitations (*Bundgaard, 1985*; *King, 1994*; *Stella, 2004; Ettmayer, 2004; Järvinen, 2005*). Prodrugs, i.e. compounds that when administered to humans or mammals by any known route, are metabolised to compounds having formula (1), belong to the invention. In particular this relates to compounds with primary or secondary amino or hydroxy groups. Such compounds can be reacted with organic acids to yield compounds having formula (1) wherein an additional group is present that is easily removed after administration, for instance, but not limited to amidine, enamine, a Mannich base, a hydroxyl-methylene derivative, an O-(acyloxymethylene carbamate) derivative, carbamate, ester, amide or enaminone.

**'Crystal form'** refers to various solid forms of the same compound, for example polymorphs, solvates and amorphous forms. **'Polymorphs'** are crystal structures in which a compound can crystallize in different crystal packing arrangements, all of which have the same elemental composition. Polymorphism is a frequently occurring phenomenon, affected by several crystallization conditions such as temperature, level of supersaturation, the presence of impurities, polarity of solvent, rate of cooling. Different polymorphs usually have different X-ray diffraction patterns, solid state NMR spectra, infrared or Raman spectra, melting points, density, hardness, crystal shape, optical and electrical properties, stability, and solubility. Recrystallization solvent, rate of crystallization, storage temperature, and other factors may cause one crystal form to dominate. **'Solvates'** are generally a crystal form that contains either stoichiometric or non-stoichiometric amounts of a solvent. Often, during the process of crystallization some compounds have a tendency to trap a fixed molar ratio of solvent molecules in the crystalline solid state, thus forming a solvate. When the solvate is water, **'hydrates'** may be formed. The compound of formula (1) and pharmaceutically acceptable salts thereof may exist in the form of a **hydrate** or a **solvate,** and such a hydrate and solvate are also encompassed in the present invention. Examples thereof include ¼ hydrate, dihydrochloride dihydrate, and the like. **'Amorphous'** forms are noncrystalline materials with no long range order, and generally do not give a distinctive powder X-ray diffraction pattern. Crystal forms in general have been described by Byrn (*1995*) and Martin (*1995*)

To provide a more concise description, some of the quantitative expressions given herein are not qualified with the term **"about".** It is understood that whether the term "about" is used explicitly or not, every quantity given herein is meant to refer to the actual given value, and it is also meant to refer to the approximation to such given value that would reasonably be inferred based on the ordinary skill in the art, including approximations due to the experimental and/or measurement conditions for such given value.

Throughout the description and the claims of this specification the word **"comprise"** and variations of the word, such as "comprising" and "comprises" is not intended to exclude other additives, components, integers or steps.

While it may be possible for the compounds of formula (1) to be administered as the raw chemical, it is preferable to present them as a **'pharmaceutical composition'.** According to a further aspect, the present invention provides a pharmaceutical composition comprising at least one compound of formula (1), at least one pharmaceutically acceptable salt or solvate thereof, or a mixture of any of the foregoing, together with one or more pharmaceutically acceptable carriers thereof, and optionally one or more other therapeutic ingredients. The carrier(s) must be 'acceptable' in the sense of being compatible with the other ingredients of the formulation and not deleterious to the recipient thereof. The term "composition" as used herein encompasses a product comprising specified ingredients in predetermined amounts or proportions, as well as any product that results, directly or indirectly, from combining specified ingredients in specified amounts. In relation to pharmaceutical compositions, this term encompasses a product comprising one or more active ingredients, and an optional carrier comprising inert ingredients, as well as any product that results, directly or indirectly, from combination, complexation or aggregation of any two or more of the ingredients, or from dissociation of one or more of the ingredients, or from other types of reactions or interactions of one or more of the ingredients. In general, pharmaceutical compositions are prepared by uniformly and intimately bringing the active ingredient into association with a liquid carrier or a finely divided solid carrier or both, and then, if necessary, shaping the product into the desired formulation. The pharmaceutical composition includes enough of the active object compound to produce the desired effect upon the progress or condition of diseases. Accordingly, the pharmaceutical compositions of the present invention encompass any composition made by admixing a compound of the present invention and a pharmaceutically acceptable carrier. By **"pharmaceutically acceptable"** it is meant the carrier, diluent or excipient must be compatible with the other ingredients of the formulation and not deleterious to the recipient thereof.

The affinity of the compounds of the invention for CB₁ receptors and their inhibition of acetylcholinesterase, were determined as described below. From the binding affinity measured for a given compound of formula (1), one can estimate a theoretical lowest effective **dose.** At a concentration of the compound equal to twice the measured Kᵢ-value, nearly 100% of the CB₁ receptors will be occupied by the compound. At a concentration of the compound equal to twice the measured inhibition constant, nearly 100% of the acetylcholinesterase will be occupied by the compound. By converting those concentrations to mg of compound per kg of patient one obtains a theoretical lowest effective dose, assuming ideal bioavailability. Pharmacokinetic, pharmacodynamic, and other considerations may alter the dose actually administered to a higher or lower value. The typical daily dose of the active ingredients varies within a wide range and will depend on various factors such as the relevant indication, the route of administration, the age, weight and sex of the patient, and may be determined by a physician. In general, total daily dose administration to a patient in single or individual doses, may be in amounts, for example, from 0.001 to 10 mg/kg body weight daily, and more usually from 0.01 to 1,000 mg per day, of total active ingredients. Such dosages will be administered to a patient in need of treatment from one to three times each day, or as often as needed for efficacy, and for periods of at least two months, more typically for at least six months, or chronically.

The term **"therapeutically effective amount"** as used herein refers to an amount of a therapeutic agent to treat a condition treatable by administrating a composition of the invention. That amount is the amount sufficient to exhibit a detectable therapeutic or ameliorative response in a tissue system, animal or human. The effect may include, for example, treating the conditions listed herein. The precise effective amount for a subject will depend upon the subject's size and health, the nature and extent of the condition being treated, recommendations of the treating physician (researcher, veterinarian, medical doctor or other clinician), and the therapeutics, or combination of therapeutics, selected for administration. Thus, it is not useful to specify an exact effective amount in advance. The term **"pharmaceutically acceptable salt"** refers to those salts that are, within the scope of sound medical judgment, suitable for use in contact with the tissues of humans and lower animals without undue toxicity, irritation, allergic response, and the like, and are commensurate with a reasonable benefit/risk ratio. Pharmaceutically acceptable salts are well-known in the art. They can be prepared *in situ* when finally isolating and purifying the compounds of the invention, or separately by reacting them with pharmaceutically acceptable non-toxic bases or acids, including inorganic or organic bases and inorganic or organic acids (*Berge, 1977*). The **'free base'** form may be regenerated by contacting the salt with a base or acid, and isolating the parent compound in the conventional matter. The parent form of the compound differs from the various salt forms in certain physical properties, such as solubility in polar solvents, but otherwise the salts are equivalent to the parent form of the compound for the purposes of the present invention. **'Complex'** refers to a complex of the compound of the invention, e.g. formula (1), complexed with a metal ion, where at least one metal atom is chelated or sequestered. Complexes are prepared by methods well known in the art (*Dwyer, 1964*).

The term **"treatment"** as used herein refers to any treatment of a mammalian, for example human condition or disease, and includes: (1) inhibiting the disease or condition, i.e., arresting its development, (2) relieving the disease or condition, i.e., causing the condition to regress, or (3) stopping the symptoms of the disease. The term **'inhibit'** includes its generally accepted meaning which includes prohibiting, preventing, restraining, alleviating, ameliorating, and slowing, stopping or reversing progression, severity, or a resultant symptom. As such, the present method includes both medical therapeutic and/or prophylactic administration, as appropriate. As used herein, the term **"medical therapy"** intendeds to include prophylactic, diagnostic and therapeutic regimens carried out *in vivo* or *ex vivo* on humans or other mammals. **'Mammals'** include animals of economic importance such as bovine, ovine, and porcine animals, especially those that produce meat, as well as domestic animals, sports animals, zoo animals, and humans, the latter being preferred. The term **"subject"** as used herein, refers to an animal, preferably a mammal, most preferably a human, who has been the object of treatment, observation or experiment.

### ABBREVIATIONS

- AChE: acetylcholinesterase
- AD: Alzheimer's disease
- APT: attached proton test
- BOP: benzotriazol-1-yl-oxytris-phosphonium hexafluorophosphate
- CB₁: cannabinoid receptor subtype-1
- CB₂: cannabinoid receptor subtype-2
- CHO: Chinese Hamster Ovary (cells)
- CIP: 2-chloro-1,3-dimethylimidazolinium hexafluorophos-phate
- DCC: dicyclohexylcarbodiimide
- DIPEA: *N,N*-diisopropylethylamine
- DMAP: 4-dimethylaminopyridin
- DMSO: dimethylsulfoxide
- HEK: Human Embryonic Kidney (cells)
- HBTU: O-benzotriazol-1-yl-N, N, N', N'-tetramethyluronium hexafluorophosphate
- HOAt: N-hydroxy-7-azabenzotriazole
- m.p.: melting point *c.q*. melting range
- MS: mass spectrometry
- PET: positron emission tomography
- *p*-TsOH: paratoluene sulphonic acid
- PyAOP: 7-azabenzotriazol-1-yl-oxytris-(pyrrolidino)-phosphonium hexafluorophosphate
- PyBOP: benzotriazol-1-yl-oxytris(pyrrolidino)-phosphonium hexafluorophosphate
- SPECT: single photon emission computed tomography
- TBTU: O-(benzotriazol-1-yl)-N,N,N',N'-tetramethyluroniumtetrafluoroborate
- THF: tetrahydrofuran

### EXAMPLE. 1: ANALYTICAL METHODS

**Nuclear magnetic resonance** spectra (¹H NMR and ¹³C NMR, APT) were determined in the indicated solvent using a Bruker ARX 400 (¹H: 400 MHz, ¹³C: 100 MHz) at 300 K, unless indicated otherwise. The spectra were determined in deuterated chloroform or dichloromethane obtained from Cambridge Isotope Laboratories Ltd. Chemical shifts (δ) are given in ppm downfield from tetramethylsilane (¹H, ¹³C) or CCl₃F (¹⁹F). Coupling constants *J* are given in Hz. Peakshapes in the NMR spectra are indicated with the symbols 'q' (quartet), 'dq' (double quartet), 't' (triplet), 'dt' (double triplet), 'd' (doublet), 'dd' (double doublet), 's' (singlet), 'bs' (broad singlet) and `m' (multiplet).

**Flash chromatography** refers to purification using the indicated eluent and silica gel (either Acros: 0.030-0.075 mm or Merck silica gel 60: 0.040-0.063 mm). **Column chromatography:** Merck's silica gel 60 (0.063-0.200 mm) plates. **Melting points** were recorded on a Büchi B545 melting point apparatus. **All reactions** involving moisture sensitive compounds or conditions were carried out under an anhydrous nitrogen atmosphere. Reactions were monitored by using **thin-layer chromatography** (TLC) on silica coated plastic sheets (Merck precoated silica gel 60 F254) with the indicated eluent. Spots were visualised by UV light (254 nm) or I₂. Dichloromethane (phosphorous pentoxide and calciumhydride), tetrahydrofuran (sodium/benzophenone ketyl) and light petroleum (60-80) were distilled freshly prior to use. All other commercially available chemicals were used without further purification.

### EXAMPLE 2: GENERAL ASPECTS OF SYNTHESES

The syntheses of essential structural elements of known cannabinoid-CB₁ antagonist are described in patent applications and/or scientific literature. For instance, well-documented are the essential cannabinoid structural elements **(A^{1a})** *(*WO01070700, Lange, 2004*^{b}),* (**A**^{1b}) *(*WO03026648*),* **(A²)** *(*WO03027076*,* WO03040107*,* WO03063781, Lange, 2005*^{b} ; Dyck (2004),* **(A³)** (EP0576357, EP1150961, Lan, .1999; *Seltzman,* 1995; *Dutta, 1994 and Katoch-Rouse, 2003),* **(A⁴)** *(*WO03007887, *Plummer, 2005),* **(A⁵)** *(*WO0307069*),* **(A⁶)** *(*WO03078413*, Lange, 2005^{b}),* **(A⁷)** *(*WO2004026301, Lange, 2005*^{b} Dyck, 2004) and* **(A⁸)** *(*WO2004013120*).*

In general terms, syntheses of compounds of formula (1) wherein n = 0, can be accomplished by reacting a compound of formula A-L, wherein L represents a leaving group, with a compound of general formula B, wherein B is a nucleophile. Syntheses of compounds of formula (1) wherein n = 1, can be accomplished by reacting a compound of formula A-T-L, (L is a leaving group), with a compound of general formula B wherein B is a nucleophile. Syntheses of compounds of formula (1) wherein n = 1 can also be accomplished by reacting a compound of formula A-L, (L is a leaving group) with a compound of formula T-B wherein T is a nucleophile. Another alternative is reacting a compound of formula A-T wherein T is a nucleophile, with a compound of general formula L-B, wherein L is a leaving group.

Specific syntheses of compounds of formula (I) wherein B represents tacrine or a tacrine analog, is outlined in Scheme 2:

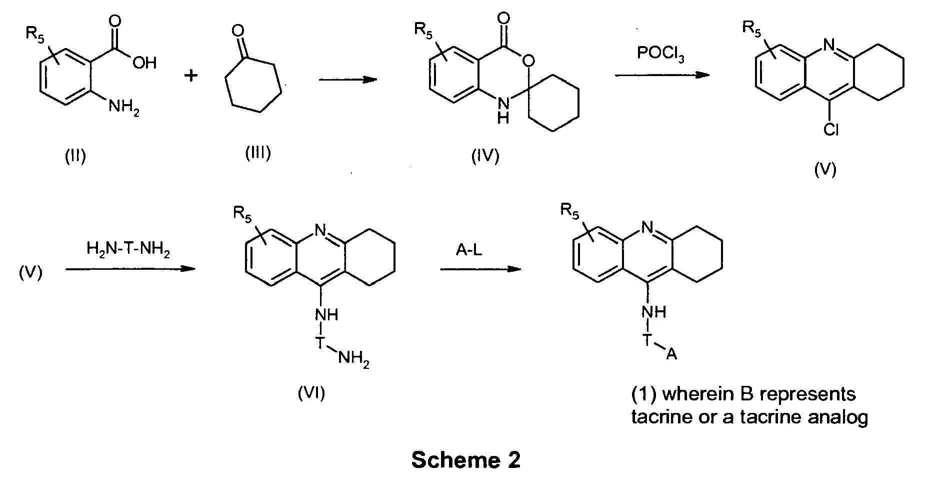

An anthranilic acid analog (II) wherein R₅ represents a hydrogen or halogen atom, or a methoxy, methyl or trifluoromethyl group, can be reacted with cyclohexanone (III) in an inert organic solvent such as toluene to give a spiro-compound of formula (IV). This compound of formula (IV) can be reacted with a chlorinating agent such as phosphorous oxychloride (POCl₃) to give a 9-chloro-1,2,3,4-tetrahydroacridine derivative (V) *(Carlier, 1999^{a})* that can be reacted with a compound of formula H₂N-T-NH₂ wherein the linker T consists of a saturated or unsaturated linear carbon chain of 2-8 atoms, which carbon chain may be substituted with 1-5 substituents selected from methyl, ethyl, hydroxy, fluoro or amino, which carbon chain may incorporate an additional nitrogen atom, optionally substituted with an C₁₋₃ alkyl group, or which carbon chain may incorporate an additional oxygen or sulphur atom or a carbonyl group or sulfonyl group or an amide (C(=O)-NH) group or a sulfonamide (S(O₂)-NH) group or a ureido group or a phenyl group or aryl group, which phenyl group or aryl group is optionally substituted with 1-4 substituents selected from halogen, cyano, methyl, methoxy, trifluoromethyl, OCHF₂, OCF₃, SCF₃ or nitro, to give a compound of general formula (VI). This reaction is preferably carried out in an inert organic solvent such as 1-pentanol at elevated temperature *(Carlier, 1999^{b}).* A compound of formula (VI) can be reacted a compound of formula A-L, wherein A represents an essential structural element of any known cannabinoid-CB₁ antagonist containing at least two phenyl rings which phenyl rings are independently optionally substituted with one or two substituents selected from halogen, methoxy and trifluoromethyl, said essential structural element being attached to a hydrogen bond acceptor in said cannabinoid-CB₁ antagonist wherein the hydrogen bond acceptor moiety represents either a carbonyl group, a sulfonyl group, or a nitrogen or oxygen atom incorporated in a heteroaromatic ring structure, and L represents a leaving group. When L represents a hydroxy group which is part of a carboxylic acid group, activating or coupling reagents may be added in order to enhance the reaction rate. *(Bodanszky, 1994; Akaji, 1994; Albericio, 1997)* This reaction can give a compound of formula (1) wherein A has the meaning as given above, T represents a linker with the abovementioned meaning, and B represent tacrine or a tacrine analog.

Syntheses of compounds of formula (1) wherein A represents the structural element (A^{1a}) or (A^{1b}),wherein R¹, R² and R³ independently represent or more hydrogen atoms, trifluoromethyl groups or halogen atoms, is outlined in Scheme 3:

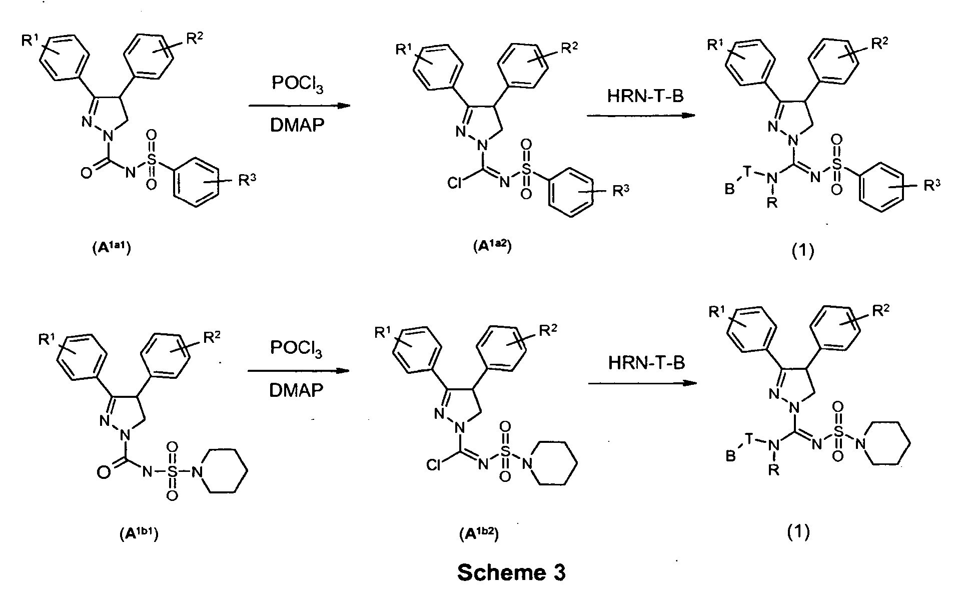

A compound of formula (A^{1a1}) can be reacted with POCl₃ in the presence of DMAP in an inert organic solvent such as CH₂Cl₂, to give the compound of formula (A^{1a2}), that can be reacted with a compound of formula HRN-T-B, wherein T represents a linker and B represents tacrine or an analog. This reaction can give a compound of formula (1) wherein A has the meaning as given above for (A^{1a}), T represents a linker, B represents tacrine or an analog, and R represents a hydrogen atom or a C₁₋₃ alkyl group. Analogously, a compound of formula (A^{1b1}) can be reacted with POCl₃ in the presence of DMAP in an inert organic solvent such as CH₂Cl₂, to give the chloride derivative of formula (A^{1b2}), that can be reacted with a compound of formula HRN-T-B wherein T represents a linker and B represent tacrine or an analog. This reaction can give a compound of formula (1) wherein A has the meaning given above for (A^{1b}), T represents a linker, B represents tacrine or an analog, and R represents hydrogen or a C₁₋₃ alkyl group.

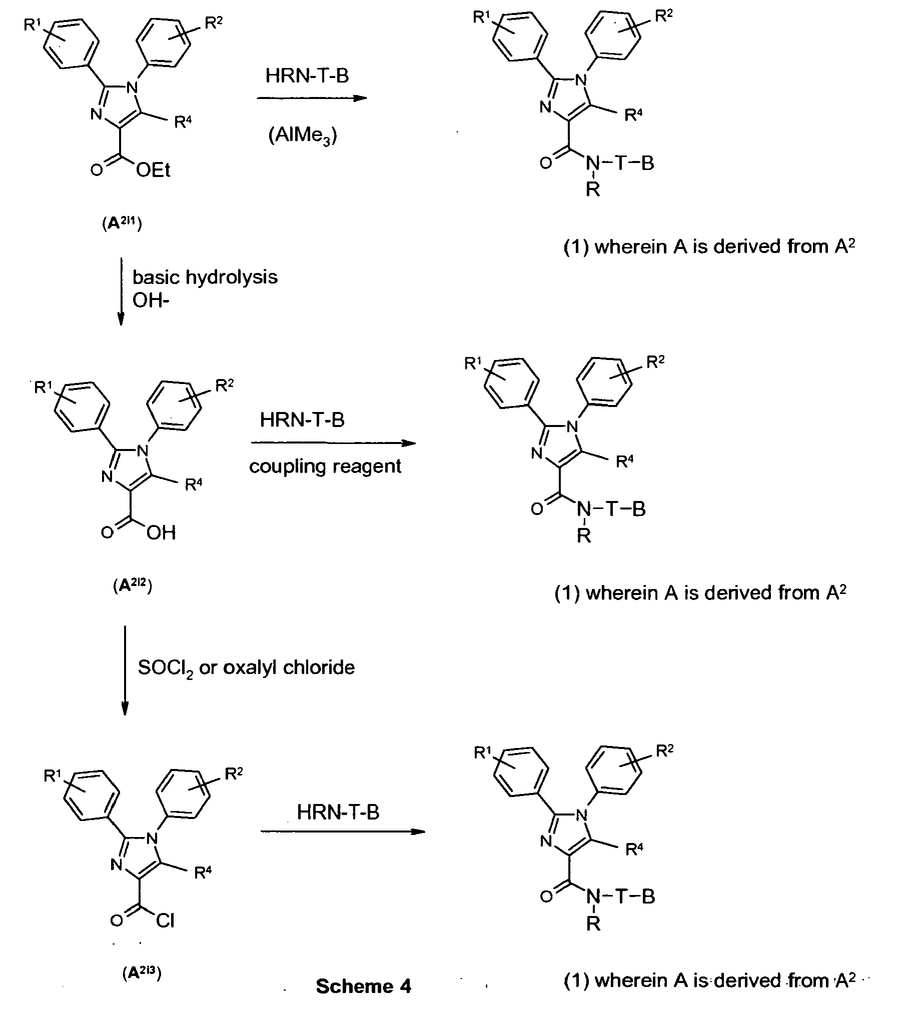

Syntheses of compounds of formula (1) wherein A represents the structural element (A²) is outlined in Scheme 4, wherein R represents a hydrogen atom or a C₁₋₃ alkyl group, R¹ and R² independently represent or more hydrogen atoms, trifluoromethyl groups or halogen atoms, R₄ represents a hydrogen or halogen atom or a methyl, ethyl, trifluoromethyl, hydroxymethyl, fluoromethyl, 2,2,2-trifluoroethyl, propyl, methyl-sulfanyl, methylsulfinyl, methylsulfonyl, ethylsulfanyl, ethylsulfinyl, ethylsulfonyl, C₁₋₃-dialkyl-aminomethyl, pyrrolidin-1-ylmethyl, piperidin-1-ylmethyl, morpholin-4-ylmethyl, and the other symbols have the meanings as given above. An ester of formula (A²ⁱ¹) can be reacted with a compound of general formula HRN-T-B to give a compound A-T-B of formula (1) wherein part A is derived from sub-structure A². Such a reaction can be catalyzed by trimethylaluminum AlMe₃ *(Levin, 1982)*.

Alternatively, a compound of formula (A²ⁱ¹) can be hydrolyzed to the corresponding carboxylic acid of formula (A²ⁱ²). A compound of formula (A²ⁱ²) can be reacted with a compound of formula HRN-T-B to give a compound of formula (1) wherein part A is derived from substructure A². This reaction preferably proceeds *via* activating and coupling methods such as formation of an active ester, or in the presence of a so-called coupling reagent, for example, DCC, HBTU, TBTU, HOAt, PyBOP, BOP, CIP, 2-chloro-1,3-dimethylimidazolinium chloride or PyAOP *(Bodanszky, 1994; Akaji, 1994; Albericio, 1997*; *Montalbetti, 2005*).Alternatively, a compound of formula (A²ⁱ²) can be converted in the presence of a chlorinating agent such as thionyl chloride or oxalyl chloride, to the corresponding acid chloride of formula (A²ⁱ³). A compound of formula (A²ⁱ³) can be reacted with a compound of formula HRN-T-B to give a compound of formula (1) wherein part A is derived from substructure A². A base like DIPEA can be added to the reaction mixture to scavenge the liberated hydrochloric acid or excess HRN-T-B can be applied for this purpose.

Analogously, the substructures of general formula (A³) (A⁴), (A⁵), (A⁶), (A⁷), or (A⁸), as shown above, can be converted into compounds A-T-B of the formula (1) wherein part A is derived from the substructures (A³) (A⁴), (A⁵), (A⁶), (A⁷), or (A⁸), respectively.

The selection of the particular synthetic procedures depends on factors known to those skilled in the art such as the compatibility of functional groups with the reagents used, the possibility to use protecting groups, catalysts, activating and coupling reagents and the ultimate structural features present in the final compound being prepared.

Pharmaceutically acceptable salts may be obtained using standard procedures well known in the art, for example by mixing a compound of the present invention with a suitable acid, for instance an inorganic acid or an organic acid.

### EXAMPLE 3: SYNTHESES OF SPECIFIC COMPOUNDS

The specific compounds of which the synthesis is described below are intended to further illustrate the invention in more detail, and therefore do not restrict the scope of the invention in any way. Other embodiments of the invention will be apparent to those skilled in the art from consideration of the specification and practice of the invention disclosed herein. It is thus intended that the specification and examples be considered as exemplary only.

### Compound 1

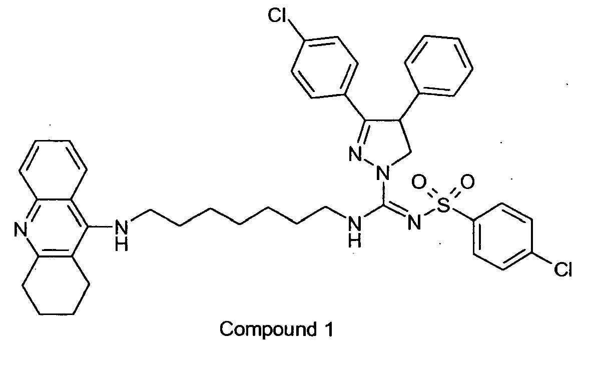

**Part A:** Spiro[2H-3,1-benzoxazine-2,1'-cyclohexan-4(1H)-one was synthesized from antranilic acid and cyclohexanone, in toluene, a in 73 % yield, as described *(Carlier, 1999^{a}).* The compound was converted to 9-chloro-1,2,3,4-tetrahydroacridine *(Carlier, 1999^{a})* in 99 % yield, and reacted with 1,2-diaminoheptane in 1-pentanol, giving N-[9'-(1',2',3',4'-tetrahydro-acridinyl)]-1,7-diaminoheptane *(Carlier, 1999^{b}).*

Analogously, N-[9'-(1',2',3',4'-tetrahydroacridinyl)]-1,7-diaminobutane was prepared from 1,2-diaminobutane and 9-chloro-1,2,3,4-tetrahydroacridine in 78 % yield. N-[9'-(1',2',3',4'-tetrahydroacridinyl)]-1,7-diaminobutane: ¹H-NMR (400 MHz, CDCl₃): δ 1.53-1.61 (m, 2H), 1.69-1.77 (m, 2H), 1.91-2.20 (m, 6H), 2.70-2.78 (m, 4H), 3.08 (br s, 2H), 3.45-3.55 (m, 2H), 4.31 (br s, 1H), 7.27-7.37 (m, 1H), 7.53-7.58 (m, 1H), 7.93-7.99 (m, 2H).

**Part B:** 3-(4-Chlorophenyl)-N-[(4-chlorophenyl)sulfonyl]-4-phenyl-4,5-dihydro-1H-pyrazole-1-carboxamide was obtained as described *(Lange, 2004^{b}).* This compound (1.5 gram, 3.16 mol) was dissolved in dichloromethane (30 ml) and DMAP (1.707 gram, 13.9 mmol) and POCl₃ 0.59 g, 3.85 mmol) were successively added and the resulting mixture was refluxed for 5 hours. The mixture was allowed to attain room temperature and was concentrated *in vacuo* to give crude 3-(4-chlorophenyl)-N-[(4-chlorophenyl)sulfonyl]-4-phenyl-4,5-dihydro-1H-pyrazole-1-carboximidoyl chloride. The obtained 3-(4-chlorophenyl)-N-[(4-chlorophenyl)sulfonyl]-4-phenyl-4,5-dihydro-1H-pyrazole-1-carboximidoyl chloride was dissolved in dichloromethane (30 ml) and reacted at 0°C with N-[9'-(1',2',3',4'-tetrahydro-acridinyl)]-1,7-diaminoheptane (1.48 gram, 4.75 mmol) and DIPEA (1.02 gram, 7.9 mmol) at reflux temperature for 72 hours. The mixture was allowed to attain room temperature and was washed with water and brine successively, dried over Na₂SO₄, filtered and concentrated. The obtained crude product was purified by flash chromatography (gradient: dichloromethane=> dichloromethane/ methanol = 95/5 (v/v)) to give pure 4-chloro-N-{[3-(4-chlorophenyl)-4-phenyl-4,5-dihydro-1H-pyrazolyl]-[7-(1,2,3,4-tetrahydroacridin-9-ylamino) heptylamino] methylene}benzene-sulfonamide (**compound 1**) (0.85 gram, 35 % yield). Melting point: 87-89 °C.

Analogously 4-chloro-N-{[3-(4-chlorophenyl)-4-phenyl-4,5-dihydro-1H-pyrazo-lyl]-[7-(1,2,3,4-tetrahydroacridin-9-yl-amino)butylamino]methylene}benzene sulfonamide **(compound 2,** m.p.: 87-89 °C). was prepared from 3-(4-chlorophenyl)-N-[(4-chloro-phenyl)sulfonyl]-4-phenyl-4,5-dihydro-1H-pyrazole-1-carboxamide and N-[9'-(1',2',3',4'-tetrahydro-acridinyl)]-1,7-diaminobutane.

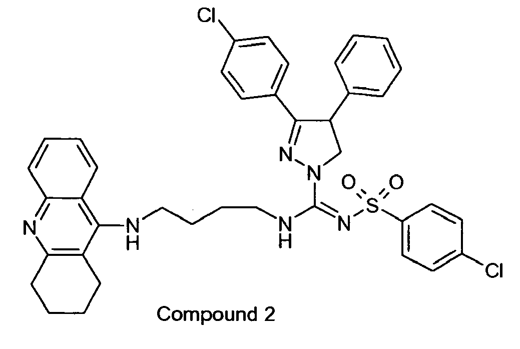

### Compound 3

**Part A:** Ethyl 2-(2-chlorophenylyl-(4-chlorophenyl)-5-ethyl-1H-imidazole-4-carboxylate was obtained according to WO03040107. To a magnetically stirred solution of ethyl 2-(2-chlorophenyl)-1-(4-chlorophenyl)-5-ethyl-1H-imidazole-4-carboxylate (5.80 g, 0.0149 mol) in tetrahydrofuran (40 ml) was added a solution of LiOH (0.715 g) in water (40 ml). The resulting mixture was heated at 70°C for 16 hours. The resulting mixture was allowed to attain room temperature and subsequently treated with concentrated hydrochloric acid (3.5 ml). The tetrahydrofuran was evaporated in vacuo and the resulting mixture was stirred overnight. The formed precipitate was collected by filtration and washed with petrolaum ether (40-60) to give 2-(2-chlorophenyl)-1-(4-chlorophenyl)-5-ethyl-1H-imidazole-4-carboxylic acid (4.52 gram, 84 % yield). ¹H-NMR (400 MHz, CDCl₃): δ 1.09 (t, J = 7, 3H), 2.90 (q, J = 7, 2H), 3.70 (br s, 1H), 7.12 (dt, J = 8 and 2, 2H), 7.22-7.28 (m, 1H), 7.29-7.38 (m, 5H).

**Part B:** To a magnetically stirred solution of N-[9'-(1',2',3',4'-tetrahydro-acridinyl)]-1,7-diaminoheptane (3.25 g, 10.4 mmol) in dichloromethane (50 ml) was successively added 2-(2-chlorophenyl)-1-(4-chlorophenyl)-5-ethyl-1H-imidazole-4-carboxylic acid (2.8 gram, 7.8 mmol), HOAt (1.3 gram, 9.4 mmol) and 1-(3-dimethylaminopropyl)-3-ethylcarbodiimide hydrochloride (1.8 gram, 9.4 mmol). The resulting mixture was stirred at room temperature for 60 hours and successively washed with water (2x 100 ml) and brine (100 ml). The organic layer was successively dried over Na₂SO₄, filtered and concentrated. The obtained crude product was purified by flash chromatography (gradient: dichloromethane/ethanol = 99/1 => dichloromethane/methanol = 90/10 (v/v)) to give pure N-[7-(1,2,3,4-tetrahydroacridin-9-ylamino)heptyl]-2-(2-chlorophenyl)-1-(4-chlorophenyl)-5-ethyl-1H-imidazole-4-carboxamide **(compound 3)** (2.25 g, 53 % yield). Melting point: 143-145 °C.

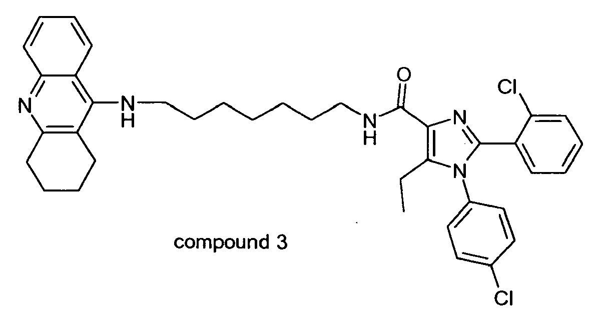

Analogously N-[4-(1,2,3,4-tetrahydroacridin-9-ylamino)butyl]-2-(2-chlorophenyl) -1-(4-chlorophenyl)-5-ethyl-1H-imidazole-4-carboxamide **(compound 4,** m.p.:103-105 °C). was prepared from 2-(2-chlorophenyl)-1-(4-chlorophenyl)-5-ethyl-1H-imidazole-4-carboxylic acid and N-[9'-(1',2',3',4'-tetrahydroacridinyl)]-1,7-diaminobutane.

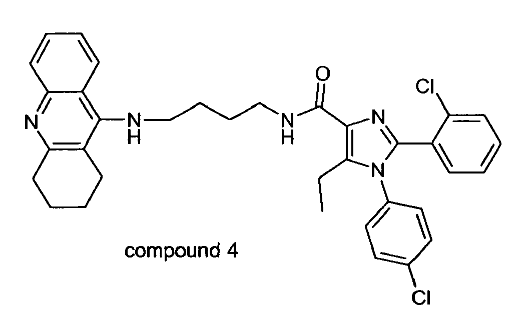

### EXAMPLE 4: FORMULATIONS

**For oral (*p.o*.) administration:** to the desired quantity (0.5-5 mg) of compound 1 in a glass tube, some glass beads were added and the solid was milled by vortexing for 2 min. After addition of 1 ml of a solution of 1% methylcellulose in water and 2% (v/v) Poloxamer 188 (Lutrol F68), the compound was suspended by vortexing for 10 min. The pH was adjusted to 7 with a few drops of aqueous NaOH (0.1N). Remaining particles in the suspension were further suspended by using an ultrasonic bath.

**For intraperitoneal (*i.p*.) administration:** to the desired quantity (0.5-15 mg) of the solid compound 1 in a glass tube, some glass beads were added and the solid was milled by vortexing for 2 minutes. After addition of 1 ml of a solution of 1% methylcellulose and 5% mannitol in water, the compound was suspended by vortexing for 10 minutes. Finally the pH was adjusted to 7.

### EXAMPLE 5: PHARMACOLOGICAL METHODS

***In vitro* affinity for human cannabinoid-CB₁ receptors** was determined using membrane preparations of CHO cells in which the human cannabinoid CB₁ receptor is stably transfected, and [³H]CP-55,940 as radioligand. After incubation of a freshly prepared cell membrane preparation with the [³H]-ligand, with or without addition of compounds of the invention, separation of bound and free ligand was performed by filtration over glassfiber filters. Radioactivity on the filter was measured by liquid scintillation counting.

**Inhibition of acetylcholinesterase in human HEK-293 cells.** Compounds were dissolved in DMSO (10 mM) and diluted to test concentrations in assay buffer. Testing was at a 3 log concentration range around a predetermined IC₅₀ for the respective assay: e.g. 10, 1, 0.1 and 0.01 µM for IC₅₀ of 0.3 µM and 300, 30, 3, and 0.3 nM for one with IC₅₀ of 10 nM. All determinations were performed as duplicates. The highest concentration tested for primes was 10 µM. Following incubation of test compound with an acetylcholinesterase enzyme preparation (human recombinant expressed in HEK-293 cells) and the substrate acetylthiocholine (50 µM) for 30 minutes at 37°C, the thio-conjugate product was determined by photometry.
Results were expressed as % of total product formed, at each concentration tested (duplicates); from the concentration - production inhibition curves IC₅₀ values were determined by non-linear regression analysis using Hill equation curve fitting. Results were expressed as plC₅₀'s. Compounds with no significant affinity at concentrations of 10 µM and higher were considered inactive: pIC₅₀ <5.0. (Ellman, 1961)

### EXAMPLE 6: PHARMACOLOGICAL TESTRESULTS

CB₁ receptor affinity data and acetylcholine esterase inhibition data obtained according to the protocols given above are shown in the table below.

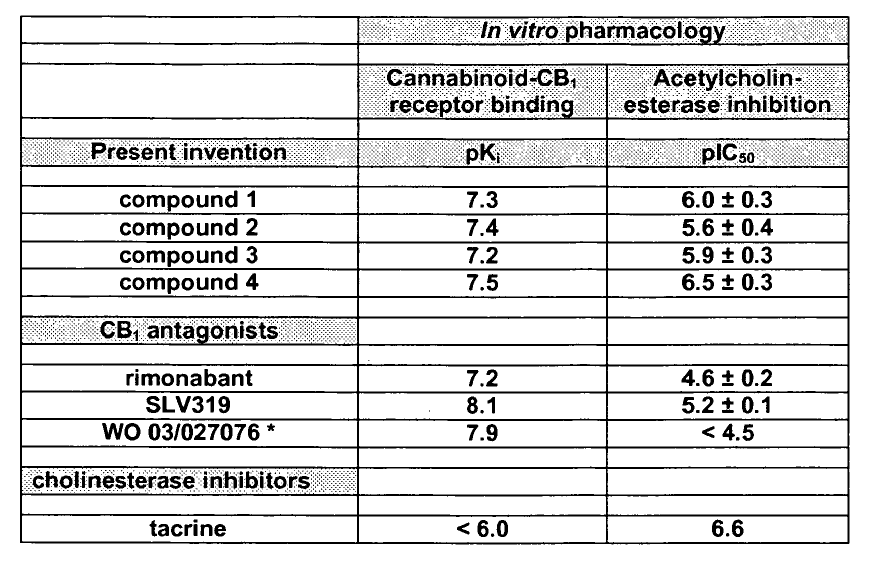

The results clearly indicate that the compounds of the invention have affinity for cannabinoid-CB₁ receptors and cholinesterase inhibiting activity. Their affinity is as potent as that of rimonabant, whilst e.g. compound 4 simultaneously is as potent a cholinesterase inhibitor as tacrine. This in sharp contrast with for instance a structurally closely related potent CB₁ antagonist disclosed in WO 03/027076 (see structures below) which is completely inactive as cholinesterase inhibitor.

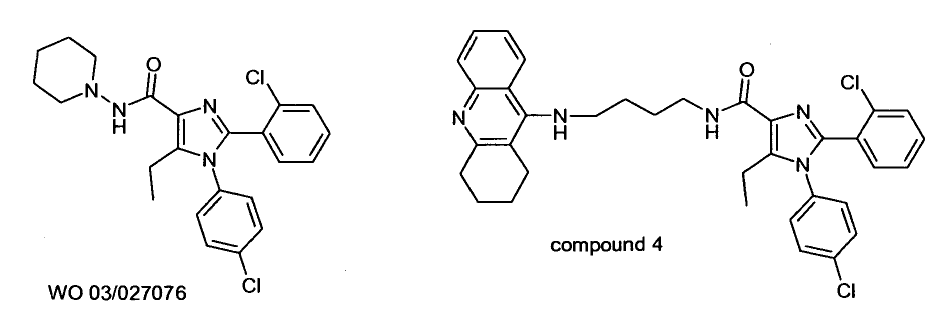

### EXAMPLE 7: PHARMACEUTICAL PREPARATIONS

For clinical use, compounds of formula (1) are formulated into pharmaceutical compositions that are important and novel embodiments of the invention because they contain the compounds, more particularly specific compounds disclosed herein. Types of pharmaceutical compositions that may be used include, but are not limited to, tablets, chewable tablets, capsules (including microcapsules), solutions, parenteral solutions, ointments (creams and gels), suppositories, suspensions, and other types disclosed herein, or are apparent to a person skilled in the art from the specification and general knowledge in the art. The active ingredient for instance, may also be in the form of an inclusion complex in cyclodextrins, their ethers or their esters. The compositions are used for oral, intravenous, subcutaneous, tracheal, bronchial, intranasal, pulmonary, transdermal, buccal, rectal, parenteral or other ways to administer. The pharmaceutical formulation contains at least one compound of formula (1) in admixture with at least one pharmaceutically acceptable adjuvant, diluent and/or carrier. The total amount of active ingredients suitably is in the range of from about 0.1 % (w/w) to about 95% (w/w) of the formulation, suitably from 0.5% to 50% (w/w) and preferably from 1% to 25% (w/w). In some embodiments, the amount of active ingredient is greater than about 95% (w/w) or less than about 0.1% (w/w).

The compounds of the invention can be brought into forms suitable for administration by means of usual processes using auxiliary substances such as liquid or solid, powdered ingredients, such as the pharmaceutically customary liquid or solid fillers and extenders, solvents, emulsifiers, lubricants, flavorings, colorings and/or buffer substances. Frequently used auxiliary substances include magnesium carbonate, titanium dioxide, lactose, saccharose, sorbitol, mannitol and other sugars or sugar alcohols, talc, lactoprotein, gelatin, starch, amylopectin, cellulose and its derivatives, animal and vegetable oils such as fish liver oil, sunflower, groundnut or sesame oil, polyethylene glycol and solvents such as, for example, sterile water and mono- or polyhydric alcohols such as glycerol, as well as with disintegrating agents and lubricating agents such as magnesium stearate, calcium stearate, sodium stearyl fumarate and polyethylene glycol waxes. The mixture may then be processed into granules or pressed into tablets. A tablet is prepared using the ingredients below:

| Ingredient | Quantity (mg/tablet) |
|---|---|
| COMPOUND No. 1 | 10 |
| Cellulose, microcrystalline | 200 |
| Silicon dioxide, fumed | 10 |
| Stearic acid | 10 |
| Total | 230 |

The components are blended and compressed to form tablets each weighing 230 mg.

The active ingredients may be separately premixed with the other non-active ingredients, before being mixed to form a formulation. The active ingredients may also be mixed with each other, before being mixed with the non-active ingredients to form a formulation.

Soft gelatin capsules may be prepared with capsules containing a mixture of the active ingredients of the invention, vegetable oil, fat, or other suitable vehicle for soft gelatin capsules. Hard gelatin capsules may contain granules of the active ingredients. Hard gelatin capsules may also contain the active ingredients together with solid powdered ingredients such as lactose, saccharose, sorbitol, mannitol, potato starch, corn starch, amylopectin, cellulose derivatives or gelatin.

Dosage units for rectal administration may be prepared (i) in the form of suppositories that contain the active substance mixed with a neutral fat base; (ii) in the form of a gelatin rectal capsule that contains the active substance in a mixture with a vegetable oil, paraffin oil or other suitable vehicle for gelatin rectal capsules; (iii) in the form of a ready-made micro enema; or (iv) in the form of a dry micro enema formulation to be reconstituted in a suitable solvent just prior to administration.

Liquid preparations may be prepared in the form of syrups, elixirs, concentrated drops or suspensions, e.g. solutions or suspensions containing the active ingredients and the remainder consisting, for example, of sugar or sugar alcohols and a mixture of ethanol, water, glycerol, propylene glycol and polyethylene glycol. If desired, such liquid preparations may contain coloring agents, flavoring agents, preservatives, saccharine and carboxymethyl cellulose or other thickening agents. Liquid preparations may also be prepared in the form of a dry powder, reconstituted with a suitable solvent prior to use. Solutions for parenteral administration may be prepared as a solution of a formulation of the invention in a pharmaceutically acceptable solvent. These solutions may also contain stabilizing ingredients, preservatives and/or buffering ingredients. Solutions for parenteral administration may also be prepared as a dry preparation, reconstituted with a suitable solvent before use.

Also provided according to the invention are formulations and 'kits of parts' comprising one or more containers filled with one or more of the ingredients of a pharmaceutical composition of the invention, for use in medical therapy. Associated with such container(s) can be written materials such as instructions for use, or a notice in the form prescribed by a governmental agency regulating the manufacture, use or sale of pharmaceuticals products, which notice reflects approval by the agency of manufacture, use, or sale for human or veterinary administration. The use of formulations of the invention in the manufacture of medicaments for treating a condition wherein antagonism of CB₁ receptors and/or inhibition of acetylcholine-esterase is required or desired, and methods of medical treatment or comprising the administration of a therapeutically effective total amount of at least one compound of formula (1), either as such or, in the case of prodrugs, after administration, to a patient suffering from, or susceptible to, a condition wherein antagonism of CB₁ receptors and/or inhibition of acetylcholinesterase is required or desired

By way of example and not of limitation, several pharmaceutical compositions are given, comprising preferred active compounds for systemic use or topical application. Other compounds of the invention or combinations thereof, may be used in place of (or in addition to) said compounds. The concentration of the active ingredient may be varied over a wide range as discussed herein. The amounts and types of ingredients that may be included are well known in the art.

### BIBLIOGRAPHY

Akaji, K. et al., Tetrahedron Lett. (1994), 35, 3315-3318
Albericio, F. et al., Tetrahedron Lett. (1997), 38, 4853-4856
Berge, S.M.: "Pharmaceutical salts", J. Pharmaceutical Science, 66, 1-19 (1977).
Berger, C. et al., J. Neurochem. 2004, 88, 1159-1167
Bickel, M.H., : "The pharmacology and Biochemistry of N-oxides", Pharmacological Reviews, 21 (4), 325 - 355, 1969.
Bodanszky, M. and A. Bodanszky: The Practice of Peptide Synthesis, Springer-Verlag, New York, 1994; ISBN: 0-387-57505-7
Brufani M. et al., Drugs of the Future 1997, 22, 397-410
Bundgaard, H. (editor), "Design of Prodrugs", Elsevier, 1985.
Byrn et al., Pharmaceutical Research, 12(7), 945-954, 1995.
Carlier, P.R. et al., Bioorg. Med. Chem. 1999a, 7, 351-357
Carlier, P.R. et al., J. Med. Chem. 1999b, 42, 4225-4231
Castellano, C. et al., Curr. Drug Targets, CNS Neurol. Disorders, 2003, 2, 389-402
Cohen, C. et al., Behav. Pharmacol. 2002, 13, 451-463
Darvesh, S. et al., Nature Rev. Neurosci. 2003, 4, 131-138
De Groot, A. et al., Mol. Pharmacol. 2006, 70, 1236-1245
De Petrocellis, L. et al,. Br. J. Pharmacol. 2004, 141, 765-774.
Di Marzo, V. et al., Nature Rev. Drug Discov. 2004, 3, 771-784.
Dutta, A. K. et al., Med. Chem. Res. 1994, 5, 54-62
Dwyer & Meilor,: "Chelating agents and Metal Chelates", Academic Press, chapter 7, 1964.
Dyck, B. et al., Bioorg. Med. Chem. Lett. 2004, 14, 1151-1154
Ellman, G.L., Courtney, K.D., Andres, V. and Featherstone, R.M. (1961) A new and rapid colorimetric determination of acetylcholinesterase activity. Biochem. Pharmacol., 7 : 88-95***.***
Ettmayer, P. et al., "Lessons learned from marketed and investigational prodrugs", J.Med.Chem., 47, 2393-2404, 2004.
Hertzog, D.L. Expert Opin. Ther. Patents 2004, 14, 1435-1452;
Hikida, T. et al., PNAS, 2003, 100, 6169-6173
Hungund, B.L. et al., Alcohol Clin. Exp. Res. 2002, 26, 565-574
Järvinen, T. et al., "Design and Pharmaceutical applications of prodrugs", pages 733-796 in: S.C. Gad (editor): "Drug Discovery Handbook", John Wiley & Sons Inc., New Jersey, U.S.A., 2005.
Katoch-Rouse, R. et al., J. Med. Chem. 2003, 46, 642-645
King, F.D., (editor), page 215 in: "Medicinal Chemistry: Principles and Practice", 1994, ISBN 0-85186-494-5.
Kumar, V. et al., Eur J Neurol 7 (2000), pp. 159-169
Lambert, D.M. and Fowler, C.J. J. Med. Chem. 2005, 48, 5059-5087;
Lan, R. et al., J. Med. Chem. 1999, 42, 769-776
Landsman, R.S. et al., Eur. J. Pharmacol. 1997, 334, R1-R2
Lange, J.H.M. and Kruse, C.G., C. Curr. Opin. Drug Discovery Dev. 2004, 7, 498-506
Lange, J.H.M. et al., J. Med. Chem. 2004b, 47, 627-643
Lange, J.H.M. and Kruse, C.G. Drug Discov. Today 2005, 10, 693-702;
Lange, J.H.M. et al., J. Med. Chem. 2005b, 48, 1823-1838
Levin, J.I., E. Turos and S. M. Weinreb, Synth. Commun., 12, 989-993, 1982**.**
Lichtman, A.H. et al., Prostaglandins Leukotrienes and Essential Fatty Acids 2002, 66, 269-285
Marco, J.L. and Carreras, M.C., Mini-Rev. Med. Chem. 2003, 3, 518-514
Martin, E.W. (Editor), "Remington: The Science and Practice of Pharmacy", Mack Publishing Company, 19th Edition, Easton, Pa, Vol 2., Chapter 83, 1447-1462, 1995.
Masanic, C.A. et al., Arch Phys Med Rehabil 82 (2001), 896-901
McKeith, I. et al., Lancet 356 (2000), 2031-2036
Montalbetti, C. and V. Falque, Tetrahedron , 61, 10827-10852, 2005
Muccioli, G.G. et al., Curr. Med. Chem. 2005, 12, 1361-1394;
Padgett, L.W. Life Sciences 2005, 77, 1767-1798*;*
Plummer, C.W. et al., Bioorg. Med. Chem. Lett. 2005, 15, 1441-1446
Racchi, M. et.al., Pharmacol. Res. 2004, 50, 441-451).
Reggio, P.H., Curr. Pharm. Des. 2003, 9, 1607-1633
Seltzman, H.H. et al., J. Chem. Soc. Chem. Commun. 1995, 1549-1550
Smith, R.A. and Fathi, Z. IDrugs 2005, 8, 53-66;
Solinas, M. et al., J. Pharmacol. Exp. Ther. 2003, 306, 93-102
Spencer, C.M. and Noble, S. Drugs Aging 13 (1998), 391-411
Stella,J., "Prodrugs as therapeutics", Expert Opin. Ther. Patents, 14(3), 277-280, 2004.
Terry, A.V. and Buccafusco, J.J., J. Pharmacol. Exp. Ther. 2003, 306, 821-827
Thakur, G.A. et al., Mini-Rev. Med. Chem. 2005, 5, 631-640;
Vandevoorde, S. & Lambert, D.M. Curr. Pharm. Des. 2005,11, 2647-68
Weinstock, M. CNS Drugs 1999, 12, 307
Werber, E.A. and Rabey, J.M., J Neural Transm 108 (2001), 1319-1325)
Wolff, M.C. and Leander, J.D., Eur. J. Pharmacol. 2003, 477, 213-217

## Claims

1. A compound having formula (1): 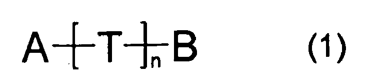 or a tautomer, stereoisomer, N-oxide, isotopically-labelled analogue, or a pharmacologically acceptable salt, hydrate or solvate of any of the foregoing, wherein:
- A represents one of the fragments (A^{1a}), (A^{1b}), (A²), (A³) (A⁴), (A⁵), (A⁶), (A⁷), or (A⁸), 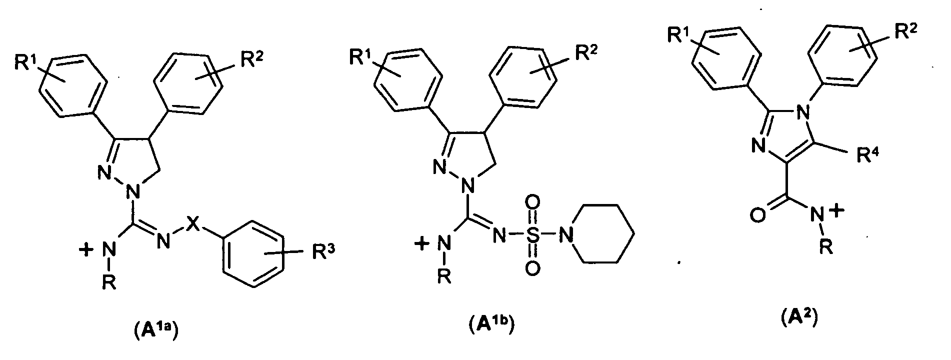 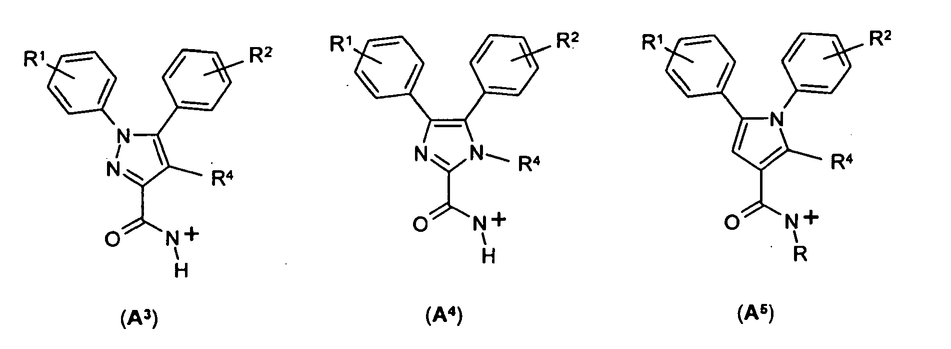 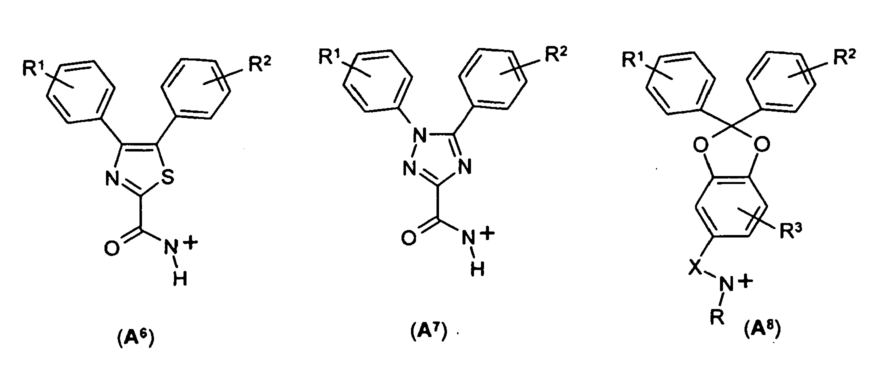 wherein, X represents a sulfonyl or a carbonyl group, the sign + represents the point at which the fragment is attached to the linker T of formula (1), R¹, R² and R³ independently represent or more hydrogen atoms, trifluoromethyl groups or halogen atoms, R₄ represents a hydrogen or halogen atom or a methyl, ethyl, trifluoromethyl, hydroxymethyl, fluoromethyl, 2,2,2-trifluoroethyl, propyl, methylsulfanyl, methylsulfinyl, methylsulfonyl, ethylsulfanyl, ethylsulfinyl, ethylsulfonyl, C₁₋₃-dialkyl-aminomethyl, pyrrolidin-1-ylmethyl, piperidin-1-ylmethyl, morpholin-4-ylmethyl, and wherein R represents a hydrogen atom or a C₁₋₃ alkyl group,
- T represents a linker consisting of a saturated or unsaturated linear carbon chain of 2-8 atoms, which carbon chain may be substituted with 1-5 substituents selected from methyl, ethyl, hydroxy, fluoro or amino, which carbon chain may contain an additional nitrogen atom, optionally substituted with a C₁₋₃ alkyl group, or which carbon chain may contain an additional oxygen or sulphur atom, or a carbonyl, sulfonyl, amide, sulfonamide, ureido, or aryl group, which aryl group is optionally substituted with 1-4 substituents selected from the group consisting of halogen, cyano, methyl, methoxy, trifluoromethyl, OCHF₂, OCF₃, SCF₃ or nitro,
- B is chosen from one of the fragments (B¹), (B²) or (B³): 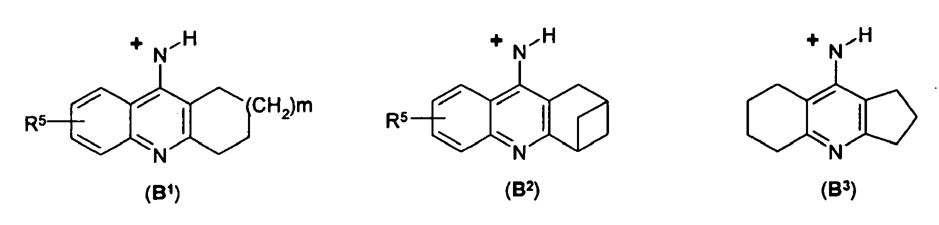 wherein, the "+" symbol represents the point at which the fragment is attached to the linker T of formula (1), R⁵ represents a hydrogen or a halogen atom, or a methoxy or a trifluoromethoxy group, and m is an integer which can have the value 0, 1 or 2,
- n = 0 or 1

2. A compound as claimed in claim 1 of formula (1) wherein: A represents one of the fragments (A^{1a}), (A^{1b}) or (A²), 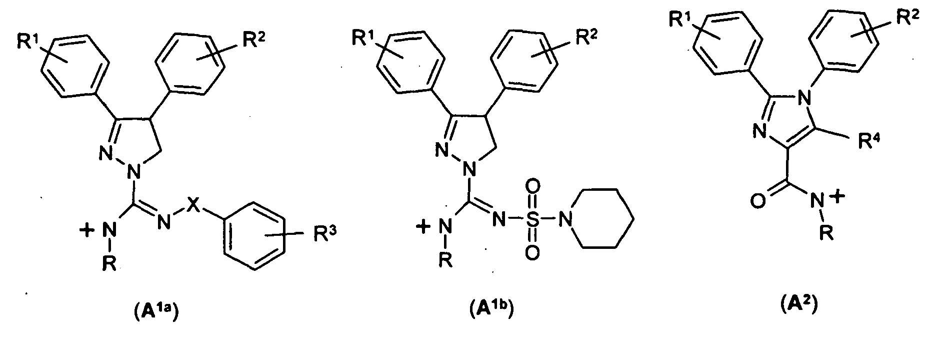 wherein, X, R, R¹, R², R³ and T have the meanings as given in claim 1, n=1 and B represents the tacrine fragment:

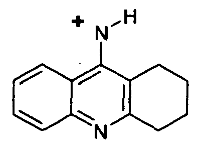

3. A compound as claimed in claim 1 of formula (1) wherein: A represents one of the fragments (A⁹) or (A¹⁰): 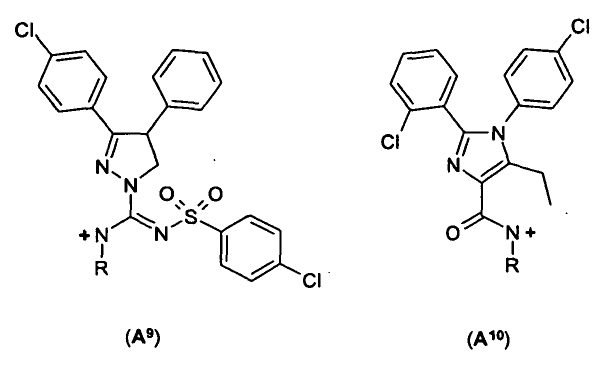 and the other symbols have the meanings as given in claim 1.

4. A compound as claimed in claim 1 selected from:
4-chloro-N-{[3-(4-chlorophenyl)-4-phenyl-4,5-dihydro-1H-pyrazolyl]-[7-(1,2,3,4-tetrahydroacridin-9-ylamino)heptylamino]methylene}benzene-sulfonamide
4-chloro-N-{[3-(4-chlorophenyl)-4-phenyl-4,5-dihydro-1H-pyrazo-lyl]-[7-(1,2,3,4-tetrahydroacridin-9-yl-amino)butylamino]methylene}benzene sulfonamide
N-[7-(1,2,3,4-tetrahydroacridin-9-ylamino)heptyl]-2-(2-chlorophenyl)-1-(4-chlorophenyl)-5-ethyl-1H-imidazole-4-carboxamide
N-[4-(1,2,3,4-tetrahydroacridin-9-ylamino)butyl]-2-(2-chlorophenyl)-1-(4-chlorophenyl)-5-ethyl-1H-imidazole-4-carboxamide

5. A compound as claimed in claim 1 selected from:

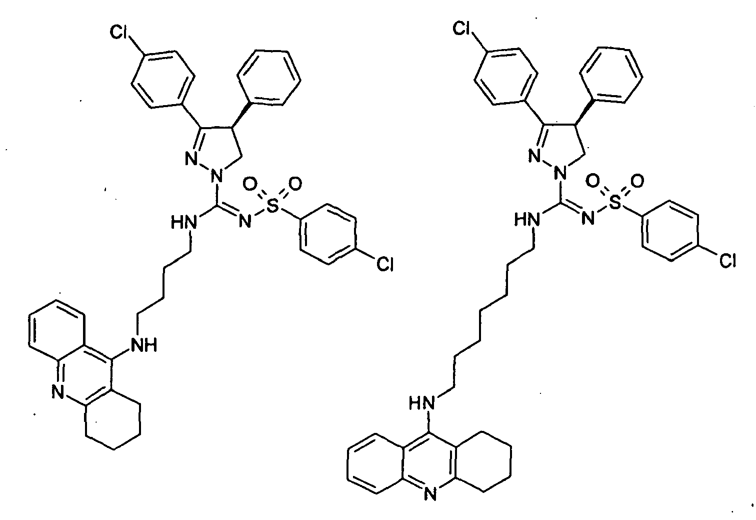

6. A compound as claimed in any of the claims 1-5. or a tautomer, stereo-isomer, N-oxide, isotopically-labelled analogue, or a pharmacologically acceptable salt, hydrate or solvate of any of the foregoing, said compound being an optically active enantiomer.

7. Process to prepare compounds as claimed in claim 1, of formula (1) wherein A represents the structural elements (A^{1a}) or (A^{1b}) wherein R¹, R² and R³ independently represent or more hydrogen atoms, trifluoromethyl groups or halogen atoms, 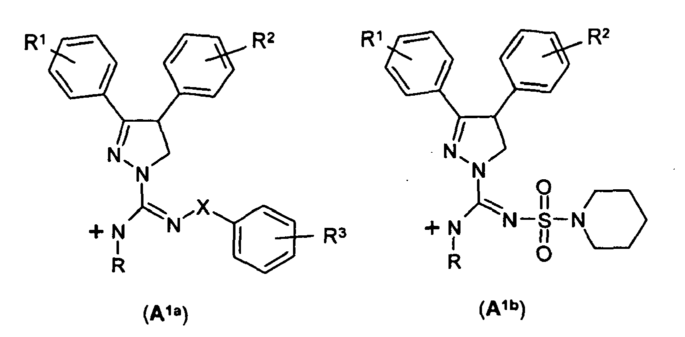 **characterized in that**: a compound of general formula (A^{1a1}) or (A^{1b1}) is reacted with a chlorinating agent, in the presence of a base, in an inert organic solvent to give the corresponding derivative of general formula (A^{1a2)} or (A^{1b2}), which can be reacted with a compound of general formula HRN-T-B wherein B represents tacrine or a tacrine analog, T has the meanings as given in claim 1, and R represents a hydrogen atom or a C₁₋₃ alkyl group, schematically:

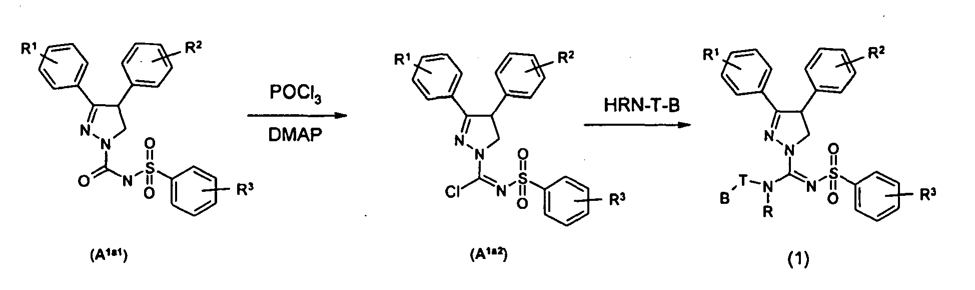

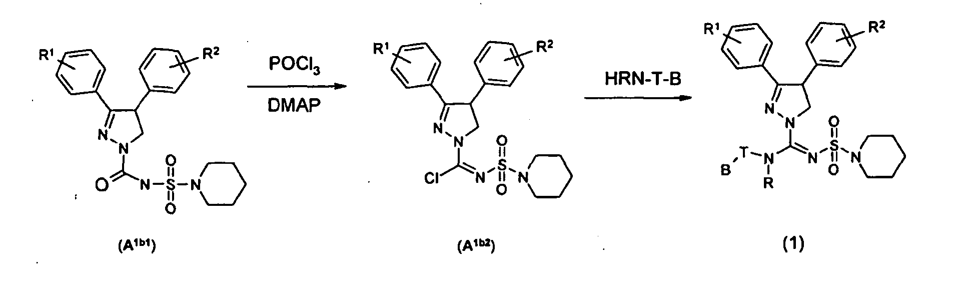

8. A compound obtainable by the process as claimed in claim 7.

9. A medicament, comprising a compound according to one of the claims 1-6, or a pharmacologically acceptable salt, hydrate, solvate, complex or conjugate thereof.

10. A pharmaceutical composition comprising, at least one pharmaceutically acceptable carrier, at least one pharmaceutically acceptable auxiliary substance, or a combination of two or more thereof; and a pharmacologically active amount of at least one compound of any one of the claims 1-6, or a pharmacologically acceptable salt, hydrate or solvate thereof.

11. The pharmaceutical composition according to claim 10, further comprising: at least one additional therapeutic agent.

12. Use of a compound as claimed in any of the claims 1-6, to prepare a pharmaceutical composition for treating addiction, appetence, alcoholism, Alzheimers disease, amnesia, anxiety, appetite disorders, arthritis, attention deficits, cancer, cardiovascular disorders, central nervous system disease, cerebral apoplexy, cerebral ischaemia, cognitive disorder, constipation, dementia, demyelinisation related disorders, depression, diabetes, diarrhoea, drug dependence, dyspepsia, dystonia, emesis, epilepsy, gastric motility disorder, gastric ulcers, gastrointestinal disorders, gastroparesis, glaucoma, Huntington's disease, impulse control disorders, irritable bowel syndrome, memory disorders, migraine, multiple sclerosis, muscle disease, muscular dystrophy, muscle spasticity, myasthenia gravis, nausea, neurodegenerative disorders, neuroinflammatory disorders, neuropathic pain, nicotine dependence, obesity, pain disorders, Parkinson's disease, *Pediculus capitis* infestation, plaque sclerosis, poison intoxication, postviral fatigue syndrome, psychiatric disorder, psychosis, senile dementia, septic shock, sexual disorders, schistosomiasis, spinal cord injury, stroke, Tourette's syndrome, traumatic brain injury, tremor, urinary dysfunction, viral encephalitis and xerostomia.

13. Use as claimed in claim 12 to prepare a pharmaceutical composition for treating Alzheimer's disease, cognitive disorders; memory disorders, dementia, attention deficits, traumatic brain injury, drug dependence, addiction and substance abuse.

14. A compound as claimed in any of the claims 1-6, for use in the treatment of addiction, appetence, alcoholism, Alzheimers disease, amnesia, anxiety, appetite disorders, arthritis, attention deficits, cancer, cardiovascular disorders, central nervous system disease, cerebral apoplexy, cerebral ischaemia, cognitive disorder, constipation, dementia, demyelinisation related disorders, depression, diabetes, diarrhoea, drug dependence, dyspepsia, dystonia, emesis, epilepsy, gastric motility disorder, gastric ulcers, gastrointestinal disorders, gastroparesis, glaucoma, Huntington's disease, impulse control disorders, irritable bowel syndrome, memory disorders, migraine, multiple sclerosis, muscle disease, muscular dystrophy, muscle spasticity, myasthenia gravis, nausea, neurodegenerative disorders, neuroinflammatory disorders, neuropathic pain, nicotine dependence, obesity, pain disorders, Parkinson's disease, *Pediculus capitis* infestation, plaque sclerosis, poison intoxication, postviral fatigue syndrome, psychiatric disorder, psychosis, senile dementia, septic shock, sexual disorders, schistosomiasis, spinal cord injury, stroke, Tourette's syndrome, traumatic brain injury, tremor, urinary dysfunction, viral encephalitis and xerostomia.

15. A compound as claimed in any of the claims 1-6, for use in the treatment of Alzheimer's disease, cognitive disorders, memory disorders, dementia, attention deficits, traumatic brain injury, drug dependence, addiction and substance abuse.

## Patentansprüche

1. Verbindung mit der Formel (1): 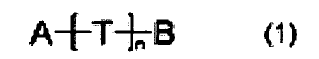 oder ein Tautomer, Stereoisomer, N-Oxid, isotopisch markiertes Analogon, oder ein pharmakologisch akzeptables Salz, Hydrat oder Solvat eines der vorgenannten Stoffe, wobei:
- A eines der Fragmente (A^{1a}) , (A^{1b}) , (A²) , (A³) , (A⁴) , (A⁵), (A⁶) , (A⁷) oder (A⁸) darstellt, 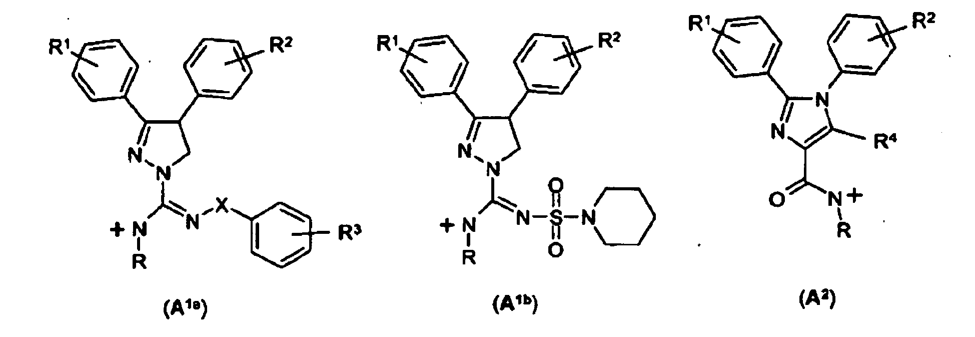 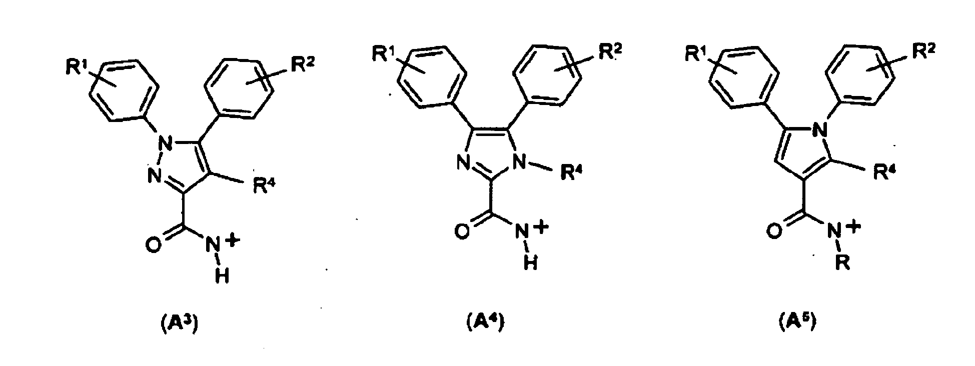 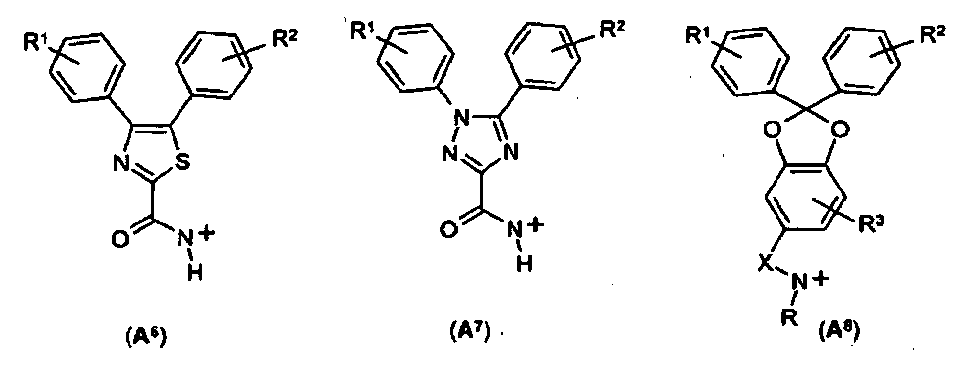 wobei X eine Sulfonyl- oder eine Carbonylgruppe darstellt, das Zeichen + den Punkt darstellt, an welchem das Fragment an den Linker T von Formel (1) gebunden ist, R¹, R² und R³ unabhängig voneinander ein(e) oder mehrere Wasserstoffatome, Trifluormethylgruppen oder Halogenatome darstellen, R₄ ein Wasserstoff- oder Halogenatom oder eine Methyl-, Ethyl-, Trifluormethyl-, Hydroxymethyl, Fluormethyl-, 2,2,2-Trifluorethyl-, Propyl-, Methylsulfanyl-, Methylsulfinyl-, Methylsulfonyl-, Ethylsulfanyl-, Ethylsulfinyl-, Ethylsulfonyl-, C_{1,3}-Dialkylaminomethyl-, Pyrrolidin-1-ylmethyl-, Piperidin-1-ylmethyl-, Morpholin-4-ylmethylgruppe darstellt, und wobei R ein Wasserstoffatom oder eine C_{1,3}-Alkylgruppe darstellt,
- T einen Linker darstellt, der aus einer gesättigten oder ungesättigten linearen Kohlenstoffkette von 2-8 Atomen besteht, wobei diese Kohlenstoffkette mit 1-5 Substituenten substituiert sein kann, die aus Methyl, Ethyl, Hydroxy, Fluor oder Amino ausgewählt sind, wobei diese Kohlenstoffkette ein zusätzliches Stickstoffatom enthalten kann, das gegebenenfalls mit einer C_{1,3}-Alkylgruppe substituiert ist, oder wobei diese Kohlenstoffkette ein zusätzliches Sauerstoff- oder Schwefelatom oder eine Carbonyl-, Sulfonyl-, Amid-, Sulfonamid-, Ureido- oder Arylgruppe enthalten kann, wobei diese Arylgruppe gegebenenfalls mit 1-4 Substituenten substituiert ist, die aus der Gruppe ausgewählt sind, die aus Halogen, Cyan, Methyl, Methoxy, Trifluormethyl, OCHF₂, OCF₃, SCF₃ und Nitro besteht,
- B aus den Fragmenten (B¹) , (B²) oder (B³) ausgewählt ist: 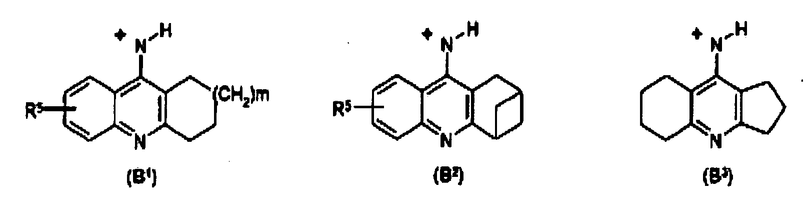 wobei das Symbol "+" den Punkt darstellt, an welchem das Fragment an den Linker T von Formel (1) gebunden ist, R⁵ ein Wasserstoff- oder ein Halogenatom oder eine Methoxy- oder eine Trifluormethoxygruppe darstellt und m eine ganze Zahl ist, welche den Wert 0, 1 oder 2 haben kann,
- n = 0 oder 1 ist.

2. Verbindung nach Anspruch 1 mit der Formel (1), wobei: A eines der Fragmente (A^{1a}) , (A^{1b}) oder (A²) darstellt, 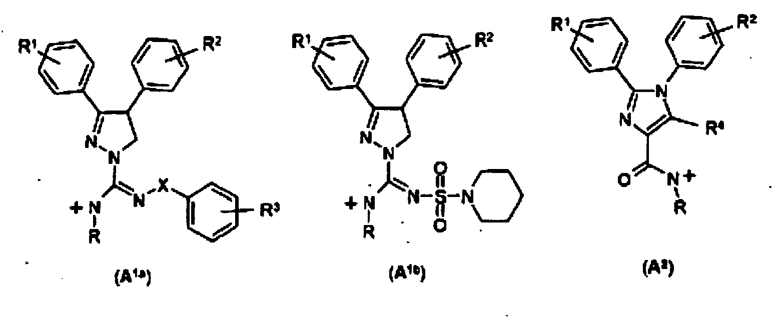 wobei X, R, R¹, R², R³ und T die in Anspruch 1 angegebenen Bedeutungen haben, n = 1 ist und B das Tacrin-Fragment darstellt:

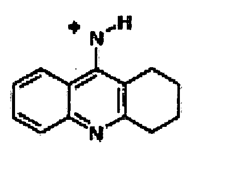

3. Verbindung nach Anspruch 1 mit der Formel (1), wobei: A eines der Fragmente (A⁹) oder (A¹⁰) darstellt: 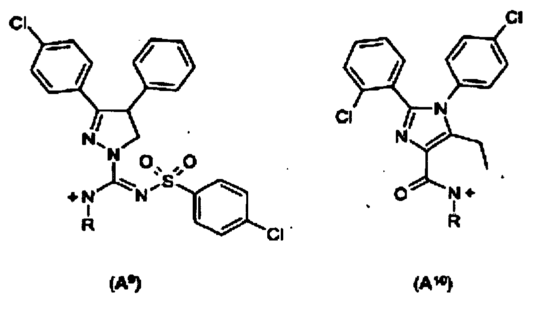 und die anderen Symbole die in Anspruch 1 angegebenen Bedeutungen haben.

4. Verbindung nach Anspruch 1, ausgewählt aus:
4-Chlor-N-{[3-(4-chlorphenyl)-4-phenyl-4,5-dihydro-1H-pyrazolyl]-[7-(1,2,3,4-tetrahydroacridin-9-ylamino)heptylamino]methylen}benzolsulfonamid,
4-Chlor-N-{[3-(4-chlorphenyl)-4-phenyl-4,5-dihydro-1H-pyrazolyl]-[7-(1,2,3,4-tetrahydroacridin-9-ylamino)butylamino]methylen}benzolsulfonamid,
N-[7-(1,2,3,4-tetrahydroacridin-9-ylamino)heptyl]-2-(2-chlorphenyl)-1-(4-chlorphenyl)-5-ethyl-1H-imidazol-4-carboxamid,
N-[4-(1,2,3,4-tetrahydroacridin-9-ylamino)butyl]-2-(2-chlorphenyl)-1-(4-chlorphenyl)-5-ethyl-1H-imidazol-4-carboxamid.

5. Verbindung nach Anspruch 1, ausgewählt aus:

6. Verbindung nach einem der Ansprüche 1-5, oder ein Tautomer, Stereoisomer, N-Oxid, isotopisch markiertes Analogon, oder ein pharmakologisch akzeptables Salz, Hydrat oder Solvat eines der vorgenannten Stoffe, wobei die Verbindung ein optisch aktives Enantiomer ist.

7. Verfahren zur Herstellung von Verbindungen nach Anspruch 1 mit der Formel (1), wobei A die Strukturelemente (A^{1a}) oder (A^{1b}) darstellt, wobei R¹, R² und R³ unabhängig voneinander ein(e) oder mehrere Wasserstoffatome, Trifluormethylgruppen oder Halogenatome darstellen, 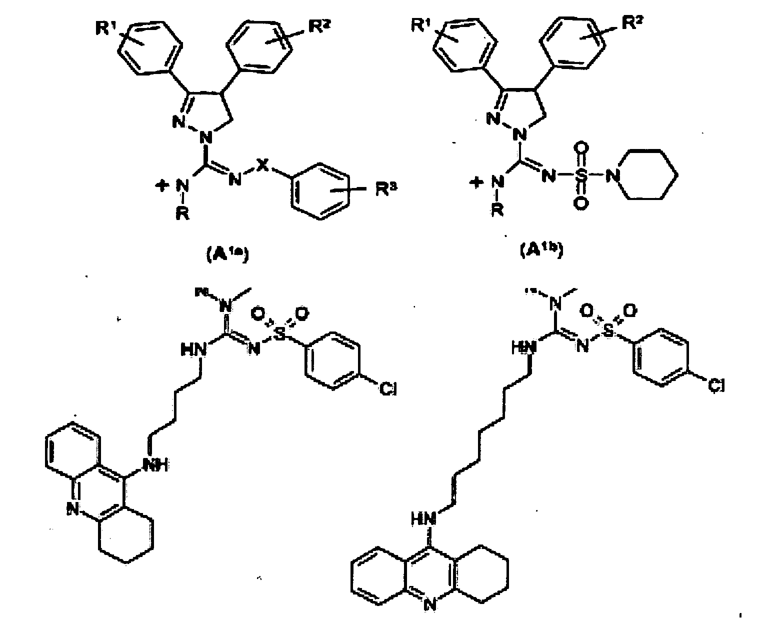 **dadurch gekennzeichnet, dass**: eine Verbindung mit der allgemeinen Formel (A^{1a1}) oder (A^{1b1}) mit einem Chlorierungsmittel in Gegenwart einer Base in einem inerten organischen Lösungsmittel umgesetzt wird, um das entsprechende Derivat mit der allgemeinen Formel (A^{1a2}) oder (A^{1b2}) zu erhalten, welches mit einer Verbindung mit der allgemeinen Formel HRN-T-B umgesetzt werden kann, wobei B Tacrin oder ein Analogon zu Tacrin darstellt, T die in Anspruch 1 angegebene Bedeutung hat und R ein Wasserstoffatom oder eine C_{1,3}-Alkylgruppe darstellt, schematisch:

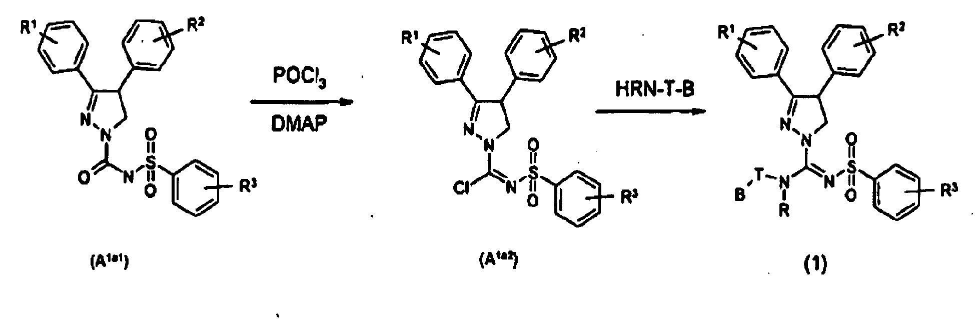

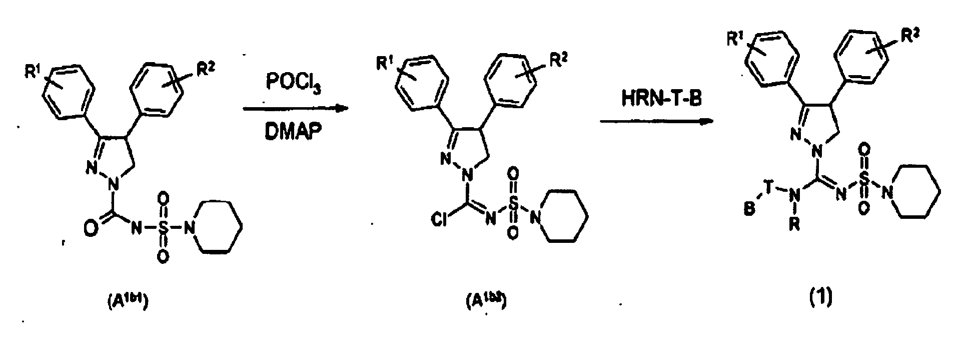

8. Verbindung, die durch das Verfahren nach Anspruch 7 herstellbar ist.

9. Arzneimittel, eine Verbindung nach einem der Ansprüche 1-6 oder ein pharmakologisch akzeptables Salz, Hydrat, Solvat, einen Komplex oder ein Konjugat davon umfasst.

10. Pharmazeutische Zusammensetzung, umfassend mindestens einen pharmazeutisch akzeptablen Träger, mindestens einen pharmazeutisch akzeptablen Hilfsstoff oder eine Kombination von zweien oder mehreren davon, und eine pharmakologisch wirksame Menge mindestens einer Verbindung nach einem der Ansprüche 1-6 oder eines pharmakologisch akzeptablen Salzes, Hydrats oder Solvats davon.

11. Pharmazeutische Zusammensetzung nach Anspruch 10, ferner umfassend: mindestens ein zusätzliches therapeutisches Mittel.

12. Verwendung einer Verbindung nach einem der Ansprüche 1-6 zur Herstellung einer pharmazeutischen Zusammensetzung zur Behandlung von Sucht, Appetenz, Alkoholismus, Alzheimer-Krankheit, Amnesie, Angstgefühl, Appetitsstörungen, Arthritis, Aufmerksamkeitsdefiziten, Krebs, kardiovaskulären Störungen, Erkrankung des Zentralnervensystems, Gehirnschlag, Hirnischämie, kognitiven Störungen, Verstopfung, Demenz, mit Demyelinisation zusammenhängenden Störungen, Depression, Diabetes, Durchfall, Drogenabhängigkeit, Dyspepsie, Dystonie, Erbrechen, Epilepsie, Störung der Magenmotilität, Magengeschwüren, Magen- und Darmstörungen, Gastroparese, Glaukom, Chorea Huntington, Störungen der Impulskontrolle, Reizdarmsyndrom, Gedächtnisstörungen, Migräne, multipler Sklerose, Muskelerkrankung, Muskeldystrophie, Muskelspastik, Myasthenia gravis, Übelkeit, neurodegenerativen Störungen, neuroinflammatorischen Erkrankungen, neuropathischen Schmerzen, Nikotinabhängigkeit, Fettleibigkeit, Schmerzstörungen, Parkinson-Krankheit, Kopflausbefall, Plaques-Sklerose, Giftintoxikation, postviralem Ermüdungssyndrom, psychiatrischer Störung, Psychose, seniler Demenz, septischem Schock, sexuellen Störungen, Schistosomiasis, Rückenmarksverletzung, Schlaganfall, Tourette-Syndrom, traumatischer Gehirnverletzung, Tremor, Blasenfunktionsstörungen, viraler Enzephalitis und Xerostomie.

13. Verwendung nach Anspruch 12 zur Herstellung einer pharmazeutischen Zusammensetzung zur Behandlung von Alzheimer-Krankheit, kognitiven Störungen, Gedächtnisstörungen, Demenz, Aufmerksamkeitsdefiziten, traumatischer Gehirnverletzung, Drogenabhängigkeit, Sucht und Drogenmissbrauch.

14. Verbindung nach einem der Ansprüche 1-6 zur Verwendung bei der Behandlung von Sucht, Appetenz, Alkoholismus, Alzheimer-Krankheit, Amnesie, Angstgefühl, Appetitsstörungen, Arthritis, Aufmerksamkeitsdefiziten, Krebs, kardiovaskulären Störungen, Erkrankung des Zentralnervensystems, Gehirnschlag, Hirnischämie, kognitiven Störungen, Verstopfung, Demenz, mit Demyelinisation zusammenhängenden Störungen, Depression, Diabetes, Durchfall, Drogenabhängigkeit, Dyspepsie, Dystonie, Erbrechen, Epilepsie, Störung der Magenmotilität, Magengeschwüren, Magen- und Darmstörungen, Gastroparese, Glaukom, Chorea Huntington, Störungen der Impulskontrolle, Reizdarmsyndrom, Gedächtnisstörungen, Migräne, multipler Sklerose, Muskelerkrankung, Muskeldystrophie, Muskelspastik, Myasthenia gravis, Übelkeit, neurodegenerativen Störungen, neuroinflammatorischen Erkrankungen, neuropathischen Schmerzen, Nikotinabhängigkeit, Fettleibigkeit, Schmerzstörungen, Parkinson-Krankheit, Kopflausbefall, Plaques-Sklerose, Giftintoxikation, postviralem Ermüdungssyndrom, psychiatrischer Störung, Psychose, seniler Demenz, septischem Schock, sexuellen Störungen, Schistosomiasis, Rückenmarksverletzung, Schlaganfall, Tourette-Syndrom, traumatischer Gehirnverletzung, Tremor, Blasenfunktionsstörungen, viraler Enzephalitis und Xerostomie.

15. Verbindung nach einem der Ansprüche 1-6 zur Verwendung bei der Behandlung von Alzheimer-Krankheit, kognitiven Störungen, Gedächtnisstörungen, Demenz, Aufmerksamkeitsdefiziten, traumatischer Gehirnverletzung, Drogenabhängigkeit, Sucht und Drogenmissbrauch.

## Revendications

1. Composé présentant la formule (1) : 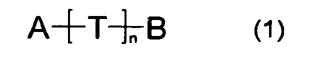 ou tautomère, stéréo-isomère, N-oxyde, analogue isotopiquement-marqué ou sel, hydrate ou solvate pharmacologiquement acceptable de l'un quelconque des précédents, dans lequel :
- A représente un des fragments (A^{1a}), (A^{1b}), (A²), (A³), (A⁴), (A⁵), (A⁶), (A⁷), ou (A⁸), 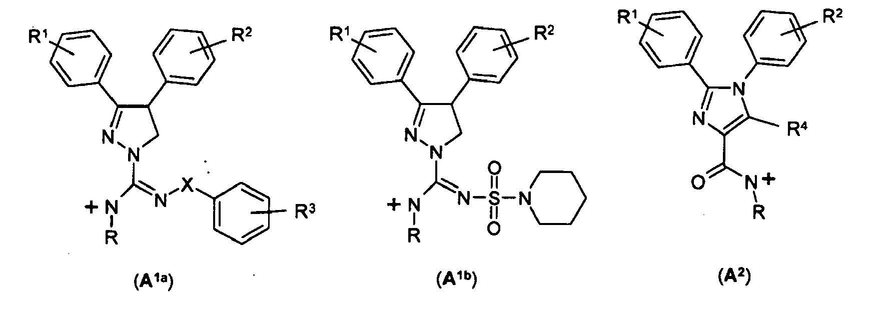 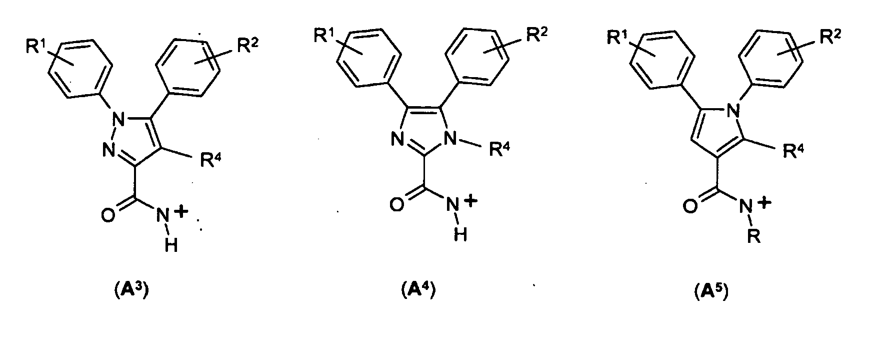 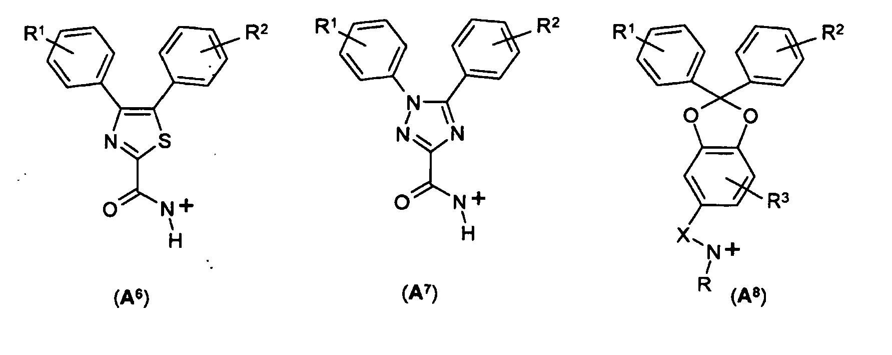 où, X représente un groupe sulfonyle ou carbonyle, le signe + représente le point auquel le fragment est fixé au coupleur T de la formule (1), R¹, R² et R³ représentent indépendamment un ou plusieurs atomes d'hydrogène, groupes trifluorométhyle ou atomes d'halogène, R⁴ représente un atome d'hydrogène ou d'halogène ou un groupe méthyle, éthyle, trifluorométhyle, hydroxyméthyle, fluorométhyle, 2,2,2-trifluoroéthyle, propyle, méthylsulfanyle, méthylsulfinyle, méthylsulfonyle, éthylsulfanyle, éthylsulfinyle, éthylsulfonyle, di(alkyle en C₁₋₃)-aminométhyle, pyrrolidin-1-ylméthyle, pipéridin-1-ylméthyle, morpholin-4-ylméthyle, et où R représente un atome d'hydrogène ou un groupe alkyle en C₁₋₃,
- T représente un coupleur consistant en une chaîne carbonée linéaire saturée ou insaturée de 2-8 atomes, laquelle chaîne carbonée peut être substituée avec 1-5 substituants choisis parmi des groupes méthyle, éthyle, hydroxy, fluoro ou amino, laquelle chaîne carbonée peut contenir un atome d'azote supplémentaire, éventuellement substitué avec un groupe alkyle en C₁₋₃, ou laquelle chaîne carbonée peut contenir un atome d'oxygène ou de soufre supplémentaire, ou un groupe carbonyle, sulfonyle, amide, sulfonamide, uréido ou aryle, lequel groupe aryle est éventuellement substitué avec 1-4 substituants choisis dans le groupe constitué d'un atome d'halogène, d'un groupe cyano, méthyle, méthoxy, trifluorométhyle, OCHF₂, OCF₃, SCF₃ ou nitro,
- B est choisi parmi un des fragments (B¹), (B²) ou (B³) : 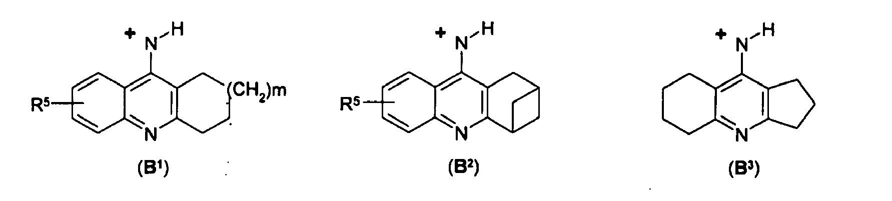 où, le symbole "+" représente le point auquel le fragment est fixé au coupleur T de la formule (1), R⁵ représente un atome d'hydrogène ou d'halogène, ou un groupe méthoxy ou trifluorométhoxy, et m est un nombre entier qui peut présenter la valeur 0, 1 ou 2,
- n = 0 ou 1.

2. Composé selon la revendication 1 de la formule (1) dans laquelle : A représente un des fragments (A^{1a}), (A^{1b}) ou (A²), 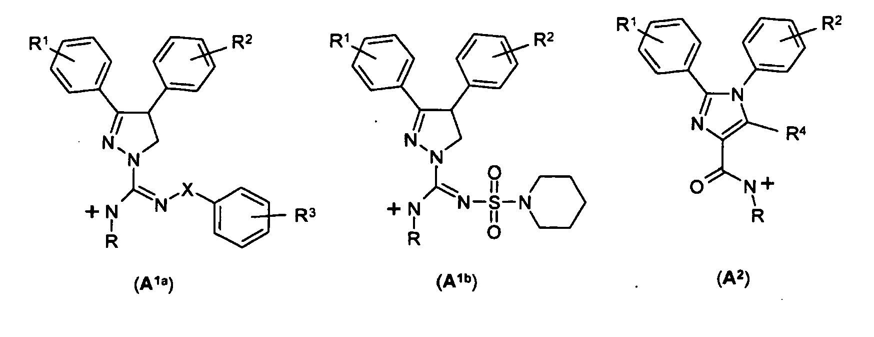 dans lesquels X, R, R¹, R², R³ et T ont les significations indiquées dans la revendication 1, n=1 et B représente le fragment de tacrine :

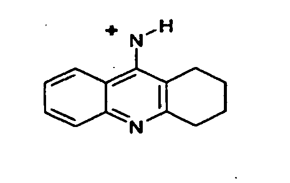

3. Composé selon la revendication 1 de la formule (1) où : A représente un des fragments (A⁹) ou (A¹⁰) : 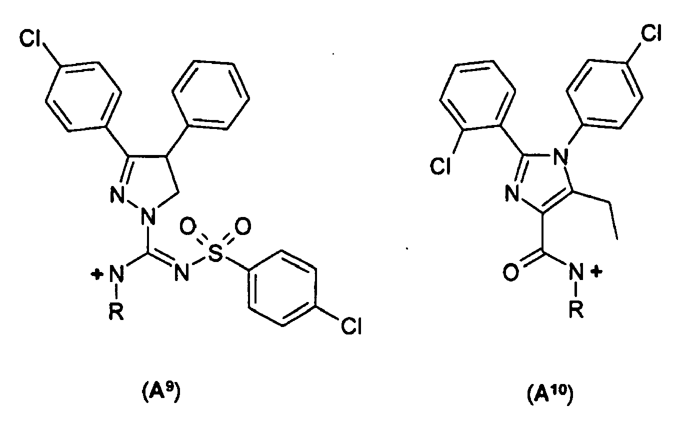 et les autres symboles ont les significations données dans la revendication 1.

4. Composé selon la revendication 1 choisi parmi :
le 4-chloro-N-{[3-(4-chlorophényl)-4-phényl-4,5-dihydro-1H-pyrazolyl]-[7-(1,2,3,4-tétrahydroacridin-9-ylamino)heptylamino]-méthylène}benzène-sulfonamide,
le 4-chloro-N-{[3-(4-chlorophényl)-4-phényl-4,5-dihydro-1H-pyrazolyl]-[7-(1,2,3,4-tétrahydroacridin-9-ylamino)butylamino]méthylène}-benzène-sulfonamide,
le N-[7-(1,2,3,4-tétrahydroacridin-9-ylamino)heptyl]-2-(2-chlorophényl)-1-(4-chlorophényl)-5-éthyl-1H-imidazole-4-carboxamide,
le N-[4-(1,2,3,4-tétrahydroacridin-9-ylamino)butyl]-2-(2-chlorophényl)-1-(4-chlorophényl)-5-éthyl-1H-imidazole-4-carboxamide.

5. Composé selon la revendication 1 choisi parmi :

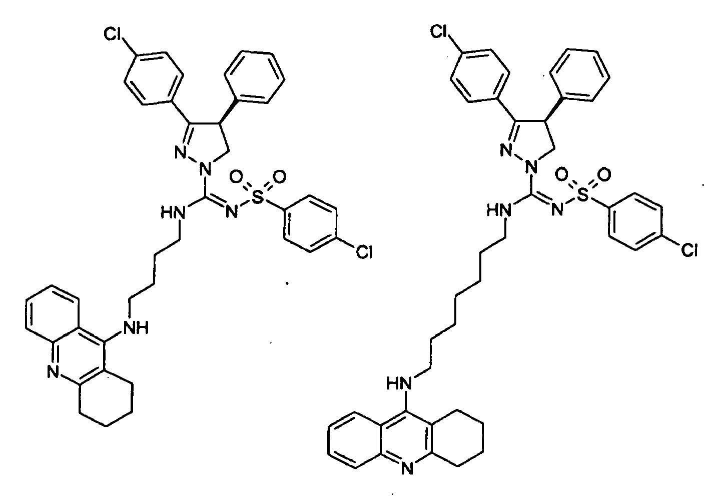

6. Composé selon l'une quelconque des revendications 1-5 ou tautomère, stéréo-isomère, N-oxyde, analogue isotopiquement-marqué ou sel, hydrate ou solvate pharmacologiquement acceptable de l'un quelconque des précédents, ledit composé étant un énantiomère optiquement actif.

7. Procédé de préparation de composés selon la revendication 1, de la formule (1) où A représente les éléments de structure (A^{1a}) ou (A^{1b}) où R¹, R² et R³ représentent indépendamment un ou plusieurs atomes d'hydrogène, groupes trifluorométhyle ou atomes d'halogène, 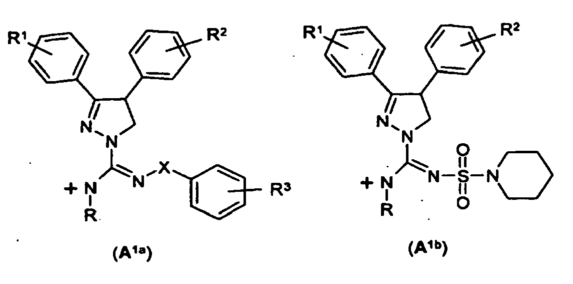 **caractérisé en ce que** : on fait réagir un composé de la formule générale (A^{1a1}) ou (A^{1b1}) avec un agent de chloration, en présence d'une base, dans un solvant organique inerte pour fournir le dérivé correspondant de formule générale (A^{1a2}) ou (A^{1b2}), lequel peut réagir avec un composé de formule générale HRN-T-B où B représente la tacrine ou un analogue de tacrine, T a les significations données dans la revendication 1, et R représente un atome d'hydrogène ou un groupe alkyle en C₁₋₃, schématiquement :

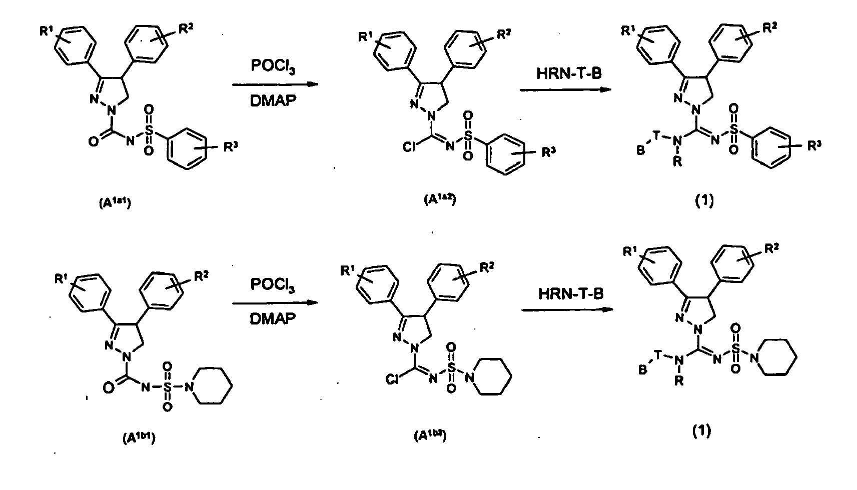

8. Composé pouvant être obtenu par le procédé selon la revendication 7.

9. Médicament comprenant un composé selon l'une quelconque des revendications 1-6, ou un sel, un hydrate, un solvate, un complexe ou un conjugué pharmacologiquement acceptable de celui-ci.

10. Composition pharmaceutique comprenant au moins un support pharmaceutiquement acceptable, au moins une substance auxiliaire pharmaceutiquement acceptable ou une combinaison de deux ou plusieurs d'entre eux ; et une quantité pharmacologiquement active d'au moins un composé selon l'une quelconque des revendications 1-6, ou un sel, un hydrate ou un solvate pharmacologiquement acceptable de celui-ci.

11. Composition pharmaceutique selon la revendication 10 comprenant de plus : au moins un agent thérapeutique supplémentaire.

12. Utilisation d'un composé selon l'une quelconque des revendications 1-6 pour préparer une composition pharmaceutique destinée au traitement de l'addiction, de l'appétence, de l'alcoolisme, de la maladie d'Alzheimer, de l'amnésie, de l'anxiété, des troubles de l'appétit, de l'arthrite, des déficits de l'attention, du cancer, des troubles cardiovasculaires, des maladies du système nerveux central, de l'apoplexie cérébrale, de l'ischémie cérébrale, des troubles cognitifs, de la constipation, de la démence, des troubles liés à la démyélinisation, de la dépression, des diabètes, des diarrhées, de la pharmacodépendance, de la dyspepsie, de la dystonie, des vomissements, de l'épilepsie, des troubles de la mobilité gastrique, d'ulcères gastriques, des troubles gastrointestinaux, de la gastroparésie, du glaucome, de la maladie d'Huntington, des troubles du contrôle des pulsions, du colon irritable, des troubles de la mémoire, de la migraine, de la sclérose en plaques, des maladies musculaires, de la dystrophie musculaire, de la spasticité musculaire, de la myasthénie grave, des nausées, des troubles neurodégénératifs, des troubles neuroinflammatoires, de la douleur neuropathique, de la dépendance à la nicotine, de l'obésité, des troubles liés à la douleur, de la maladie de Parkinson, de l'infection par *Pediculus capitis,* de la sclérose en plaques, d'une intoxication par un poison, du syndrome de fatigue post-virale, des troubles psychiatriques, de la psychose, de la démence sénile, du choc septique, des troubles sexuels, de la schistosomiase, des lésions de la moelle épinière, de l'attaque, du syndrome de la Tourette, des lésions traumatiques cérébrales, des tremblements, des dysfonctionnements urinaires, de l'encéphalite virale et de la xérostomie.

13. Utilisation selon la revendication 12 pour la préparation d'une composition pharmaceutique destinée au traitement de la maladie d'Alzheimer, des troubles cognitifs, des troubles de la mémoire, de la démence, des déficits de l'attention, des lésions traumatiques cérébrales, de la pharmacodépendance, de l'addiction et de l'usage de substances toxiques.

14. Composé selon l'une quelconque des revendications 1-6, destiné à l'utilisation dans le traitement de l'addiction, de l'appétence, de l'alcoolisme, de la maladie d'Alzheimer, de l'amnésie, de l'anxiété, des troubles de l'appétit, de l'arthrite, des déficits de l'attention, du cancer, des troubles cardiovasculaires, des maladies du système nerveux central, de l'apoplexie cérébrale, de l'ischémie cérébrale, des troubles cognitifs, de la constipation, de la démence, des troubles liés à la démyélinisation, de la dépression, des diabètes, des diarrhées, de la pharmacodépendance, de la dyspepsie, de la dystonie, des vomissements, de l'épilepsie, des troubles de la mobilité gastrique, d'ulcères gastriques, des troubles gastrointestinaux, de la gastroparésie, du glaucome, de la maladie d'Huntington, des troubles du contrôle des pulsions, du colon irritable, des troubles de la mémoire, de la migraine, de la sclérose en plaques, des maladies musculaires, de la dystrophie musculaire, de la spasticité musculaire, de la myasthénie grave, des nausées, des troubles neurodégénératifs, des troubles neuroinflammatoires, de la douleur neuropathique, de la dépendance à la nicotine, de l'obésité, des troubles liés à la douleur, de la maladie de Parkinson, de l'infection par *Pediculus capitis,* de la sclérose en plaques, d'une intoxication par un poison, du syndrome de fatigue post-virale, des troubles psychiatriques, de la psychose, de la démence sénile, du choc septique, des troubles sexuels, de la schistosomiase, des lésions de la moelle épinière, de l'attaque, du syndrome de la Tourette, des lésions traumatiques cérébrales, des tremblements, des dysfonctionnements urinaires, de l'encéphalite virale et de la xérostomie.

15. Composé selon l'une quelconque des revendications 1-6 destiné à être utilisé dans le traitement de la maladie d'Alzheimer, des troubles cognitifs, des troubles de la mémoire, de la démence, des déficits de l'attention, des lésions traumatiques cérébrales, de la pharmacodépendance, de l'addiction et de l'usage de substances toxiques.
